# EUROPEAN PATENT SPECIFICATION

(11) **EP 1 710 577 B1**
(45) Date of publication and mention of the grant of the patent: **21.07.2010**
(21) Application number: 06008833.3
(22) Date of filing: 21.12.2001
(51) Int. Cl.: G01N 33/48, G06F 19/00

(54) **Rapid and quantitative proteome analysis and related methods**
Schnelle und quantitative Proteomanalyse und entsprechende Verfahren
Analyse protéomique rapide et quantitative et procédés associés

(30) Priority: 26.12.2000 US 748783; 26.12.2000 US 748793
(43) Date of publication of application: 11.10.2006
(62) Divisional of application: 01991551.1
(73) Proprietor: The Institute for Systems Biology, Seattle, WA 98103-8904 (US); University of Washington, Seattle, WA 98105-4608 (US)
(72) Inventor: Goodlett, David R., Seattle, WA 98199 (US); Aebersold, Rudolf H., CH-8049 Zürich (CH)
(74) Representative: UEXKÜLL & STOLBERG

(56) References cited:
- WO-A-99/12040
- COURCHESNE P L ET AL: "Identification of Proteins by Matrix-Assisted Laser Desorption/Ionization Mass Spectroscopy Using Peptide and Fragment Ion Masses (from 2-D Proteome Analysis Protocols, Ed. A. J. Link)" METHODS IN MOLECULAR BIOLOGY, HUMANA PRESS INC., CLIFTON, NJ, US, vol. 112, 1999, pages 487-511, XP002103063
- CORTHALS G L ET AL: "IDENTIFICATION OF PROTEINS BY MASS SPECTROMETRY" PROTEOME RESEARCH: TWO-DIMENSIONAL GEL ELECTROPHORESIS AND IDENTIFICATION METHODS, XX, XX, 1999, pages 1-17, XP002951117
- PENNINGTON S R ET AL: "Proteome analysis: from protein characterization to biological function" TRENDS IN CELL BIOLOGY, ELSEVIER SCIENCE LTD, XX, vol. 7, no. 7, April 1997 (1997-04), pages 168-173, XP002103064 ISSN: 0962-8924

### SEQUENCE LISTING

<110> The Institute for Sytems Biology
   University of washington
   Goodlett, David R.
   Aebersold, Rudolf H.
<120> Rapid and Quantitative Proteome Analysis and Related Methods
<130> 66661-135
<140> 06008833.3
   <141> 2001-12-21
<150> US 09/748,793
   <151> 2000-12-26
<150> US 09748,783
   <151> 2000-12-26
<160> 117
<170> FastSEQ for windows version 4.0
<210> 1
   <211> 8
   <212> PRT
   <213> Homo sapiens
<400> 1
   Ala Ser His Leu Gly Leu Ala Arg
    1               5
<210> 2
   <211> 8
   <212> PRT
   <213> Homo sapiens
<400> 2
   Arg Pro Pro Gly Phe Ser Pro Arg
    1               5
<210> 3
   <211> 10
   <212> PRT
   <213> Homo sapiens
<400> 3
   Ala Cys Ile Ser Glu Ile Leu Pro Ser Lys
    1               5                   10
<210> 4
   <211> 9
   <212> PRT
   <213> Homo sapiens
<400>
   Gly Val Arg Tyr Ser Phe Gly Phe Lys
   1 5
<210> 5
   <211> 9
   <212> PRT
   <213> Homo sapiens
<400> 5
   Arg Ala Asn Leu Ile Ser Gln Cys Arg
   1 5
<210> 6
   <211> 10
   <212> PRT
   <213> Homo sapiens
<400> 6
   Arg Cys Gly Leu Pro Ser Ser Gly Lys Arg
   1 5 10
<210> 7
   <211> 9
   <212> PRT
   <213> Homo sapiens
<400> 7
   Arg Asp Ile Thr Leu Glu Ala Ser Arg
   1 5
<210> 8
   <211> 8
   <212> PRT
   <213> Homo sapiens
<400> 8
   Arg Glu Arg Glu Thr Leu Glu Lys
   1 5
<210> 9
   <211> 8
   <212> PRT
   <213> Homo sapiens
<400> 9
   Arg Leu Thr Glu Glu Glu Arg Lys
   1 5
<210> 10
   <211> 9
   <212> PRT
   <213> Homo sapiens
<400> 10
   Arg Leu Val Glu Val Asp Ser Ser Arg
   1 5
<210> 11
   <211> 8
   <212> PRT
   <213> Homo sapiens
<400> 11
   Arg Asn Leu Leu Asp His His Arg
   1 5
<210> 12
   <211> 10
   <212> PRT
   <213> Homo sapiens
<400> 12
   Arg Pro His Ala Ala Gln Pro Gly Ala Arg
   1 5 10
<210> 13
   <211> 10
   <212> PRT
   <213> Homo sapiens
<400> 13
   Arg Pro Gln Thr Ala Thr Ala Ser Thr Lys
   1 5 10
<210> 14
   <211> 9
   <212> PRT
   <213> Homo sapiens
<400> 14
   Arg Arg Pro Ser Ala Tyr Gln Ala Leu
   1 5
<210> 15
   <211> 7
   <212> PRT
   <213> Homo sapiens
<400> 15
   Ala Cys Tyr Ile Lys Val Lys
   1 5
<210> 16
   <211> 7
   <212> PRT
   <213> Homo sapiens
<400> 16
   Ala Asp Pro Leu Pro Arg Arg
   1 5
<210> 17
   <211> 8
   <212> PRT
   <213> Homo sapiens
<400> 17
   Ala Phe Val Ala Phe Ala Ala Lys
   1 5
<210> 18
   <211> 7
   <212> PRT
   <213> Homo sapiens
<400> 18
   Ala Phe Val Phe Gly Arg Lys
   1 5
<210> 19
   <211> 7
   <212> PRT
   <213> Homo sapiens
<400> 19
   Ala His Ala Glu Ile Arg Lys
   1 5
<210> 20
   <211> 7
   <212> PRT
   <213> Homo sapiens
<400> 20
   Ala His Glu Ala Lys Ile Arg
   1 5
<210> 21
   <211> 7
   <212> PRT
   <213> Homo sapiens
<400> 21
   Ala Leu Glu Ala His Lys Arg
   1 5
<210> 22
   <211> 7
   <212> PRT
   <213> Homo sapiens
<400> 22
   Ala Leu Gln Phe Phe Ala Lys
   1 5
<210> 23
   <211> 7
   <212> PRT
   <213> Homo sapiens
<400> 23
   Ala Met Ala Ile Tyr Lys Lys
   1 5
<210> 24
   <211> 7
   <212> PRT
   <213> Homo sapiens
<400> 24
   Ala Pro Asp Pro Arg Leu Arg
   1 5
<210> 25
   <211> 8
   <212> PRT
   <213> Homo sapiens
<400> 25
   Ala Val Ala Gly His Leu Thr Arg
   1 5
<210> 26
   <211> 12
   <212> PRT
   <213> Saccharomyces cerevisiae
<400> 26
   Tyr Arg Pro Asn Cys Pro Ile Ile Leu Val Thr Arg
   1 5 10
<210> 27
   <211> 13
   <212> PRT
   <213> Saccharomyces cerevisiae
<400> 27
   Leu Asp Asn Cys Val Ile Asn Glu Lys Gly Ile Val Lys
   1 5 10
<210> 28
   <211> 12
   <212> PRT
   <213> Saccharomyces cerevisiae
<400> 28
   Arg Pro Met Ile Cys Leu Lys Asn Asn Ser Leu Arg
   1 5 10
<210> 29
   <211> 13
   <212> PRT
   <213> Saccharomyces cerevisiae
<400> 29
   Asn Lys Ala Asn Ile Cys Leu Ala Lys Asn Asp Leu Lys
   1 5 10
<210> 30
   <211> 12
   <212> PRT
   <213> Saccharomyces cerevisiae
<400> 30
   Tyr Leu Ile Val Leu Thr Gln Asn Cys His Leu Lys
   1 5 10
<210> 31
   <211> 12
   <212> PRT
   <213> Saccharomyces cerevisiae
<400> 31
   Phe Tyr Thr Lys Arg Leu Cys Thr Val Ser Val Lys
   1 5 10
<210> 32
   <211> 12
   <212> PRT
   <213> Saccharomyces cerevisiae
<400> 32
   Met Leu Arg Asn Leu Val Val Arg Asn Ala Cys Arg
   1 5 10
<210> 33
   <211> 14
   <212> PRT
   <213> Saccharomyces cerevisiae
<400> 33
   Gly Cys Glu Val Val Val Ser Gly Lys Leu Arg Ala Ala Arg
   1 5 10
<210> 34
   <211> 14
   <212> PRT
   <213> Saccharomyces cerevisiae
<400> 34
   Ser Ile Gln Leu Thr Leu Lys Gly Pro Ser Gly Cys Leu Lys
   1 5 10
<210> 35
   <211> 13
   <212> PRT
   <213> Saccharomyces cerevisiae
<400> 35
   Val Ile Thr Leu Glu Lys Ile Cys Glu Val Ala Ala Arg
   1 5 10
<210> 36
   <211> 12
   <212> PRT
   <213> Saccharomyces cerevisiae
<400> 36
   Ile Asp Arg Leu Leu Leu Lys Cys Asp Leu Ser Arg
   1 5 10
<210> 37
   <211> 14
   <212> PRT
   <213> Saccharomyces cerevisiae
<400> 37
   Arg Gly Thr Asp Val Leu Lys Ala Leu Cys Leu Gly Ala Lys
   1 5 10
<210> 38
   <211> 12
   <212> PRT
   <213> Saccharomyces cerevisiae
<400> 38
   Val Lys Pro Ile Arg Gly Phe Cys Gln Arg Leu Lys
   1 5 10
<210> 39
   <211> 9
   <212> PRT
   <213> Saccharomyces cerevisiae
<400> 39
   Lys His Cys Gln Ser Gln Val Ser Arg
   1 5
<210> 40
   <211> 9
   <212> PRT
   <213> Saccharomyces cerevisiae
<400> 40
   Met Met Ile Ala Phe Thr Cys Lys Lys
   1 5
<210> 41
   <211> 8
   <212> PRT
   <213> Saccharomyces cerevisiae
<400> 41
   Glu Phe Arg Phe Cys Arg Ser Lys
   1 5
<210> 42
   <211> 9
   <212> PRT
   <213> Saccharomyces cerevisiae
<400> 42
   Lys Thr Leu Met Ser Val Cys Tyr Lys
   1 5
<210> 43
   <211> 10
   <212> PRT
   <213> Saccharomyces cerevisiae
<400> 43
   Lys Cys Lys Asn Gly Pro Ser Pro Asn Lys
   1 5 10
<210> 44
   <211> 10
   <212> PRT
   <213> Saccharomyces cerevisiae
<400> 44
   Val Cys Ser Ser His Thr Gly Leu Ile Arg
   1 5 10
<210> 45
   <211> 8
   <212> PRT
   <213> Saccharomyces cerevisiae
<400> 45
   Cys Tyr Leu Gln Tyr Arg Val Lys
   1 5
<210> 46
   <211> 9
   <212> PRT
   <213> Saccharomyces cerevisiae
<400> 46
   Leu Thr Val Gln Arg Pro Gln Cys Lys
   1 5
<210> 47
   <211> 9
   <212> PRT
   <213> Saccharomyces cerevisiae
<400> 47
   Val Val Gln Leu Gln Asn Ile Cys Arg
   1 5
<210> 48
   <211> 9
   <212> PRT
   <213> Saccharomyces cerevisiae
<400> 48
   Phe Ile His Val Pro Thr Cys Lys Lys
   1 5
<210> 49
   <211> 10
   <212> PRT
   <213> Saccharomyces cerevisiae
<400> 49
   Thr Ile Gly Leu Leu Leu Cys Asp Pro Lys
   1 5 10
<210> 50
   <211> 10
   <212> PRT
   <213> Saccharomyces cerevisiae
<400> 50
   Phe Tyr Arg Cys Tyr Asn Ser Tyr Ala Arg
   1 5 10
<210> 51
   <211> 10
   <212> PRT
   <213> Saccharomyces cerevisiae
<400> 51
   Tyr Lys Asp Cys Glu Asp Trp Lys Gln Lys
   1 5 10
<210> 52
   <211> 11
   <212> PRT
   <213> Saccharomyces cerevisiae
<400> 52
   Phe Thr Asp Met Gln Leu Glu Leu Cys Ser Arg
   1 5 10
<210> 53
   <211> 11
   <212> PRT
   <213> Saccharomyces cerevisiae
<400> 53
   Met Ser Cys Glu Ser Glu Gln Phe Leu Leu Arg
   1 5 10
<210> 54
   <211> 11
   <212> PRT
   <213> Saccharomyces cerevisiae
<400> 54
   Glu Thr Tyr Glu Ile Ile Cys Asp Gln Thr Lys
   1 5 10
<210> 55
   <211> 10
   <212> PRT
   <213> Saccharomyces cerevisiae
<400> 55
   Met Ala Thr Asp Glu Arg Cys Ile Phe Arg
   1 5 10
<210> 56
   <211> 13
   <212> PRT
   <213> Saccharomyces cerevisiae
<400> 56
   Ser Tyr Ser Ser Cys Pro Ala Val Ser Gln Ser Val Lys
   1 5 10
<210> 57
   <211> 11
   <212> PRT
   <213> Saccharomyces cerevisiae
<400> 57
   Thr Ile Asp Phe Lys Phe Pro Glu Cys Asp Lys
   1 5 10
<210> 58
   <211> 13
   <212> PRT
   <213> Saccharomyces cerevisiae
<400> 58
   Asn Ala Pro Gly Asp Cys Val Ser Pro Val Gln Gln Lys
   1 5 10
<210> 59
   <211> 12
   <212> PRT
   <213> Saccharomyces cerevisiae
<400> 59
   Thr Leu Ala Cys Tyr Ser Asp Ala Ile Val Met Arg
   1 5 10
<210> 60
   <211> 13
   <212> PRT
   <213> Saccharomyces cerevisiae
<400> 60
   Gly His Asn Ile Pro Cys Thr Ser Thr Ile Ser Gly Arg
   1 5 10
<210> 61
   <211> 11
   <212> PRT
   <213> Saccharomyces cerevisiae
<400> 61
   Asp Gly Val Arg Tyr Val Ile Phe Asp Cys Arg
   1 5 10
<210> 62
   <211> 12
   <212> PRT
   <213> Saccharomyces cerevisiae
<400> 62
   Met Phe Pro Ile Gly Cys Ala Thr Phe Leu Ser Arg
   1 5 10
<210> 63
   <211> 12
   <212> PRT
   <213> Saccharomyces cerevisiae
<400> 63
   Leu Lys Gly Lys Asp Ser Phe Ser Met Val Cys Lys
   1 5 10
<210> 64
   <211> 32
   <212> PRT
   <213> Drosophila melanogaster
<400> 64
   Lys His Ile Ala Asp Leu Ala Gly Asn Lys Glu Ile Ile Leu Pro Val
   1 5 10 15
   Pro Ala Phe Asn Val Ile Asn Gly Gly Ser His Ala Gly Asn Lys Leu
   20 25 30
<210> 65
   <211> 31
   <212> PRT
   <213> Drosophila melanogaster
<400> 65
   Arg Gly Thr Leu Ile Gly His Asn Gly Trp Val Thr Gln Ile Ala Thr
   1 5 10 15
   Asn Pro Lys Asp Pro Asp Thr Ile Ile Ile Ser Ala Ser Arg Asp
   20 25 30
<210> 66
   <211> 20
   <212> PRT
   <213> Drosophila melanogaster
<400> 66
   Lys Val Asn Gln Ile Gly Thr Val Thr Glu Ser Ile Ala Ala His Leu
   1               5                   10                  15
   Leu Ala Lys Lys
               20
<210> 67
   <211> 23
   <212> PRT
   <213> Drosophila melanogaster
<400> 67
   Arg Ala Ala Asp Glu Ser Phe Lys Gly Val Thr Phe Ile Ser Pro Ala
   1               5                   10                  15
   His Val Thr Leu Pro Lys Ser
               20
<210> 68
   <211> 19
   <212> PRT
   <213> Drosophila melanogaster
<400> 68
   Lys Ala Phe Val Ala Ile Gly Asp Asn Asn Gly His Ile Gly Leu Gly
   1               5                   10                  15
   Val Lys Cys
<210> 69
   <211> 19
   <212> PRT
   <213> Drosophila melanogaster
<400> 69
   Lys Leu Asp Lys Ser Val Ile His Asp Ile Val Leu Val Gly Gly Ser
   1               5                   10                  15
   Thr Arg Ile
<210> 70
   <211> 31
   <212> PRT
   <213> Drosophila melanogaster
<400> 70
   Lys Ala Ser Ile Val Gln Gln Pro Asp Gly Gln Ser Pro Ile Ala Ala
   1               5                   10                  15
   Ile Pro Gln Leu Gln Ile Gln Pro Ser Pro Gln His Ser Arg Leu
               20                  25                  30
<210> 71
   <211> 20
   <212> PRT
   <213> Drosophila melanogaster
<400> 71
   Lys Leu His Ala Asp Phe Gln Ser Asn Pro Pro Ile Ala Gly Ser Tyr
   1               5                   10                  15
   Thr Pro Lys Arg
               20
<210> 72
   <211> 23
   <212> PRT
   <213> Drosophila melanogaster
<400> 72
   Arg Gly Val Ala Phe His Gln Gln Met Pro Leu Phe Val Ser Gly Gly
   1               5                   10                  15
   Asp Asp Tyr Lys Ile Lys Val
               20
<210> 73
   <211> 29
   <212> PRT
   <213> Drosophila melanogaster
<400> 73
   Arg Ile Asn Asn Ala Val Asn Leu Asp Tyr Thr Gln Pro Gln Ala Ala
   1               5                   10                  15
   Val Ala Ala Ala Pro His Gly Tyr Gln Pro Thr Arg Gly
               20                  25
<210> 74
   <211> 17
   <212> PRT
   <213> Drosophila melanogaster
<400> 74
   Lys Ala Asn Glu Trp Val Gln His Val Ser Ala Thr Leu Gly Gly Lys
   1               5                   10                  15
   Gly
<210> 75
   <211> 18
   <212> PRT
   <213> Drosophila melanogaster
<400> 75
   Arg Ile His Phe Pro Leu Val Thr Tyr Ala Pro Val Ile Ser Ala Glu
   1               5                   10                  15
   Lys Ala
<210> 76
   <211> 19
   <212> PRT
   <213> Drosophila melanogaster
<400> 76
   Lys Val Thr His Ala Val Val Thr Val Pro Ala Tyr Phe Asn Asp Ala
   1               5                   10                  15
   Gln Arg Gln
<210> 77
   <211> 19
   <212> PRT
   <213> Drosophila melanogaster
<400> 77
   Lys Leu Asp Lys Ser Val Ile His Asp Ile Val Leu Val Gly Gly Ser
   1               5                   10                  15
   Thr Arg Ile
<210> 78
   <211> 23
   <212> PRT
   <213> Drosophila melanogaster
<400> 78
   Arg Ala Ala Asp Glu Ser Phe Lys Gly Val Thr Phe Ile Ser Pro Ala
   1               5                   10                  15
   His Val Thr Leu Pro Lys Ser
               20
<210> 79
   <211> 16
   <212> PRT
   <213> Drosophila melanogaster
<400> 79
   Lys Ser Val Ile His Asp Ile Val Leu Val Gly Gly Ser Thr Arg Ile
   1               5                   10                  15
<210> 80
   <211> 22
   <212> PRT
   <213> Drosophila melanogaster
<400> 80
   Lys Leu Gly His Val Val Met Gly Thr Gln Pro Leu Ser Pro Tyr Gln
   1 5 10 15
   Gln Leu Val Glu Lys Ile
   20
<210> 81
   <211> 23
   <212> PRT
   <213> Drosophila melanogaster
<400> 81
   Lys Val Tyr Val Gly Asn Leu Gly Ser Ser Ala Ser Lys His Glu Ile
   1 5 10 15
   Glu Gly Ala Phe Ala Lys Tyr
   20
<210> 82
   <211> 18
   <212> PRT
   <213> Drosophila melanogaster
<400> 82
   Arg Ile His Phe Pro Leu Val Thr Tyr Ala Pro Val Ile Ser Ala Glu
   1 5 10 15
   Lys Ala
<210> 83
   <211> 20
   <212> PRT
   <213> Drosophila melanogaster
<400> 83
   Lys Leu His Ala Asp Phe Gln Ser Asn Pro Pro Ile Ala Gly Ser Tyr
   1 5 10 15
   Thr Pro Lys Arg
   20
<210> 84
   <211> 26
   <212> PRT
   <213> Drosophila melanogaster
<400> 84
   Arg Val Leu Lys Pro Pro Gly Gly Gly His Thr Asn Ile Phe Ser Glu
   1 5 10 15
   Pro Asp Val Ala Val Pro Ala Pro Arg Ala
   20 25
<210> 85
   <211> 31
   <212> PRT
   <213> Drosophila melanogaster
<400> 85
   Asp Trp Tyr Gln Gly Val Gln Gln Ile Ala Ala Lys Ser Pro Leu Leu
   1               5                   10                  15
   Ile Ser Gln Thr Ala His Lys Ser Asp Met Arg Glu Leu Asn Tyr
               20                  25                  30
<210> 86
   <211> 21
   <212> PRT
   <213> Drosophila melanogaster
<400> 86
   Lys Leu Ala Ala Ala Val Leu Gly Gly Val Glu Gln Ile His Met Pro
   1               5                   10                  15
   Pro Gly Ser Lys Val
               20
<210> 87
   <211> 26
   <212> PRT
   <213> Drosophila melanogaster
<400> 87
   Lys Ser Ile Ile Thr Leu Asp Gly Asn Lys Leu Thr Gln Glu Gln Lys
   1               5                   10                  15
   Gly Asp Lys Pro Thr Thr Ile Val Arg Glu
               20                  25
<210> 88
   <211> 22
   <212> PRT
   <213> Drosophila melanogaster
<400> 88
   Lys Leu Gly His Val Val Met Gly Thr Gln Pro Leu Ser Pro Tyr Gln
   1               5                   10                  15
   Gln Leu Val Glu Lys Ile
               20
<210> 89
   <211> 17
   <212> PRT
   <213> Drosophila melanogaster
<400> 89
   Gln Lys His Pro Glu Leu Glu Ser Ile Pro Asn Leu His Val Ile Lys
   1 5 10 15
   Ala
<210> 90
   <211> 22
   <212> PRT
   <213> Drosophila melanogaster
<400> 90
   Arg Ala Ala Tyr Leu Asn Leu Ala Gln Asp Pro Ser His Pro Ala Met
   1 5 10 15
   Ser Leu Asn Ala Arg Phe
   20
<210> 91
   <211> 20
   <212> PRT
   <213> Drosophila melanogaster
<400> 91
   Lys Leu His Ile Ile Glu Val Gly Ala Pro Pro Asn Gly Asn Gln Pro
   1 5 10 15
   Phe Ala Lys Lys
   20
<210> 92
   <211> 20
   <212> PRT
   <213> Drosophila melanogaster
<400> 92
   Lys Ser Val Gly Leu Lys Pro Asp Ile Pro Glu Asp Leu Tyr His Met
   1 5 10 15
   Ile Lys Lys Ala
   20
<210> 93
   <211> 17
   <212> PRT
   <213> Drosophila melanogaster
<400> 93
   Arg Ala Ala Pro Gln Leu Asp Leu Gly Gly Gly His Tyr Val Pro Arg
   1 5 10 15
   Gln
<210> 94
   <211> 15
   <212> PRT
   <213> Drosophila melanogaster
<400> 94
   Arg Leu Val Gln His Pro Asn Ser Tyr Phe Met Asp Val Lys Cys
   1 5 10 15
<210> 95
   <211> 17
   <212> PRT
   <213> Drosophila melanogaster
<400> 95
   Gln Lys His Pro Glu Leu Glu Ser Ile Pro Asn Leu His Val Ile Lys
   1 5 10 15
   Ala
<210> 96
   <211> 17
   <212> PRT
   <213> Drosophila melanogaster
<400> 96
   Lys Tyr Ala Phe Val Gly Asp His Ala Gly Gln Ile Thr Met Leu Arg
   1 5 10 15
   Cys
<210> 97
   <211> 16
   <212> PRT
   <213> Drosophila melanogaster
<400> 97
   Lys Ala Ile Glu Leu Ser Pro Gly Asn Ala Leu Phe His Ala Lys Arg
   1 5 10 15
<210> 98
   <211> 18
   <212> PRT
   <213> Drosophila melanogaster
<400> 98
   Lys Ala Val Leu Gly Lys Asp Glu Asp Phe Lys Gln Phe Ile Gly His
   1 5 10 15
   Lys Thr
<210> 99
   <211> 27
   <212> PRT
   <213> Drosophila melanogaster
<400> 99
   Leu Cys Lys Ser Ser Ser Lys Gly Ser Ala Ser Asn Leu Arg Cys Leu
    1               5                  10                  15
   Ile Val Arg Ser Ser Tyr Leu Tyr Met Gln Pro
               20                  25
<210> 100
   <211> 23
   <212> PRT
   <213> Drosophila melanogaster
<400> 100
   Phe Val Glu Ile Lys Lys Asn Ser Phe Arg Leu Ile Leu Met Cys Ile
    1               5                  10                  15
   Ile Leu Tyr Val Leu Ile Asn
               20
<210> 101
   <211> 19
   <212> PRT
   <213> Drosophila melanogaster
<400> 101
   Gly Thr Pro Gly Gly Ile Trp Asn Gln Leu Leu Val Asp Thr Glu Leu
    1               5                  10                  15
   Ile Pro Asn
<210> 102
   <211> 16
   <212> PRT
   <213> Drosophila melanogaster
<400> 102
   Lys Ala Asn Ala Gly Thr Phe Phe Val Ala His Asp Ile Phe Arg Leu
    1               5                  10                  15
<210> 103
   <211> 29
   <212> PRT
   <213> Drosophila melanogaster
<400> 103
   Asp Ala Ser Ser Met Ser Thr Cys Ser Trp Ser Asp Met Tyr Arg Gly
    1               5                  10                  15
   Ala Leu Gln Ser Thr Asp Gly Cys Thr Gly Met Pro Pro
               20                  25
<210> 104
   <211> 13
   <212> PRT
   <213> Drosophila melanogaster
<400> 104
   Arg Leu Asp His Lys Phe Asp Leu Met Tyr Ala Lys Arg
    1               5                  10
<210> 105
   <211> 25
   <212> PRT
   <213> Drosophila melanogaster
<400> 105
   Thr Pro Ser Asn Ser Gly Leu Ser Met His Ile Glu Gln Gly Leu Lys
    1               5                  10                  15
   Asp Ile Gln Arg Ser Ser Ala Leu Ile
               20                  25
<210> 106
   <211> 18
   <212> PRT
   <213> Drosophila melanogaster
<400> 106
   Lys Val Tyr His Pro Phe Ile Val Gly Pro Tyr Ser Glu Asn Leu Asn
    1               5                  10                  15
   Lys Leu
<210> 107
   <211> 24
   <212> PRT
   <213> Drosophila melanogaster
<400> 107
   Ser Asp Lys Ser Lys Ser Asn Ser Ser Ser Ser Gly Ser Asp Ser Ser
    1               5                  10                  15
   Ser Ser Ser Ser Ser Ser Asp Ser
               20
<210> 108
   <211> 32
   <212> PRT
   <213> Drosophila melanogaster
<400> 108
   Ser Phe Val Met Lys Pro Pro Ile Gly Pro Ile Cys Trp Pro Thr Ser
    1               5                  10                  15
   Gly Val Ser Phe Asp Arg Glu Arg Cys Leu Val Ala Gly Trp Gly Arg
               20                  25                  30
<210> 109
   <211> 20
   <212> PRT
   <213> Drosophila melanogaster
<400> 109
   Leu Phe Arg Met Val Lys His Gln Val Asp Glu Val Ala Ala Met Leu
    1               5                  10                  15
   Asn Ser His Leu
               20
<210> 110
   <211> 14
   <212> PRT
   <213> Drosophila melanogaster
<400> 110
   Lys Val His Met Val Gly Ile Asp Ile Phe Ser Asn Lys Lys
    1               5                  10
<210> 111
   <211> 15
   <212> PRT
   <213> Drosophila melanogaster
<400> 111
   Arg Leu Val Gln His Pro Asn Ser Tyr Phe Met Asp Val Lys Cys
    1               5                  10                  15
<210> 112
   <211> 35
   <212> PRT
   <213> Drosophila melanogaster
<400> 112
   Gly Gly Gly Pro Gln Pro Ala Pro Ala Gly Thr Gly Arg Pro Gly Phe
    1               5                  10                  15
   Gly Leu Gly Ile Ser Ser Thr Thr Ser Thr Thr Thr Thr Thr Ala Lys
               20                  25                  30
   Pro Ile Thr
           35
<210> 113
   <211> 32
   <212> PRT
   <213> Drosophila melanogaster
<400> 113
   Asp Arg Asp Arg Ser Arg Asp Lys Ser His Ser Lys His Ser Ser Ser
   1 5 10 15
   Ser Ser Ser Lys His Ser Ser Ser Asn Ser Ser Ser Ser Lys His Lys
   20 25 30
<210> 114
   <211> 18
   <212> PRT
   <213> Drosophila melanogaster
<400> 114
   Lys Tyr Tyr Val Thr Ile Ile Asp Ala Pro Gly His Arg Asp Phe Ile
   1 5 10 15
   Lys Asn
<210> 115
   <211> 27
   <212> PRT
   <213> Drosophila melanogaster
<400> 115
   Gln Ser Gln Arg Leu Pro Pro Glu Gln Leu Lys Leu Ile Gly Met Leu
   1 5 10 15
   Tyr Arg Lys His Leu Gln Ile Ile Leu Gln His
   20 25
<210> 116
   <211> 13
   <212> PRT
   <213> Drosophila melanogaster
<400> 116
   Arg Leu Asp His Lys Phe Asp Leu Met Tyr Ala Lys Arg
   1 5 10
<210> 117
   <211> 35
   <212> PRT
   <213> Drosophila melanogaster
<400> 117
   Trp Lys Ile Val Ser Gly Leu Thr Leu Ser Asp Phe Ala Lys Thr Lys
    1               5                  10                  15
   Leu Ser Val Thr Gly Lys Glu Leu Gln Glu Glu Lys Asp Glu Ala Leu
               20                  25                  30
   Ser Val Leu
           35

## Description

The present invention relates to a method of identifying a polypeptide.

The classical biochemical approach to study biological processes has been based on the purification to homogeneity by sequential fractionation and assay cycles of the specific activities that constitute a process, the detailed structural, functional and regulatory analysis of each isolated component, and the reconstitution of the process from the isolated components. The Human Genome Project and other genome sequencing programs are turning out in rapid succession the complete genome sequences of specific species and, thus, in principle the amino acid sequence of every protein potentially encoded by that species. It is to be expected that this information resource unprecedented in the history of biology will enhance traditional research, methods and catalyze progress in fundamentally different research paradigms, one of which is Proteomics.

Efforts to sequence the entire human genome along with the genomes of a number of other species have been extraordinarily successful. The genomes of numerous microbial species (TIGR Microbial Database; www.tigr.org) have been completed and the genomes of over one hundred twenty other microbial species are in the process of being sequenced. Additionally, the more complex genomes of eukaryotes, in particular those of the genetically well characterized unicellular organism *Saccharomyces cerevisiae* and the multicellular species *Caenorhabditis elegans* and *Drosophila melanogaster* have been sequenced completely. Furthermore, "draft sequence" of the rice genome has been published, and completion of the human and *Arabidopsis* genomes are imminent. Even in the absence of complete genomic sequences, rich DNA sequence databases have been made publicly available, including those containing over 2.1 million human and over 1.2 million murine expressed sequence tags (ESTs).

ESTs are stretches of approximately 300 to 500 contiguous nucleotides representing partial gene sequences that are being generated by systematic single pass sequencing of the clones in cDNA libraries. On the timescale of most biological processes, with the notable exception of evolution, the genomic DNA sequence can be viewed as static, and a genomic sequence database therefore represents an information resource akin to a library. Intensive efforts are underway to assign "function" to individual sequences in sequence databases. This is attempted by the computational analysis of linear sequence motifs or higher order structural motifs that indicate a statistically significant similarity of a sequence to a family of sequences with known function, or by other means such as comparison of homologous protein functions across species. Other methods have also been used to determine function of individual sequences, including experimental methods such as gene knockouts and suppression of gene expression using antisense nucleotide technology, which can be time consuming and in some cases still insufficient to allow assignment of a biological function to a polypeptide encoded by the sequence.

The proteome has been defined as the protein complement expressed by a genome. This somewhat restrictive definition implies a static nature of the proteome. In reality the proteome is highly dynamic since the types of expressed proteins, their abundance, state of modification, and subcellular locations are dependent on the physiological state of the cell or tissue. Therefore, the proteome can reflect a cellular state or the external conditions encountered by a cell, and proteome analysis can be viewed as a genome-wide assay to differentiate and study cellular states and to determine the molecular mechanisms that control them. Considering that the proteome of a differentiated cell is estimated to consist of thousands to tens of thousands of different types of proteins, with an estimated dynamic range of expression of at least 5 orders of magnitude, the prospects for proteome analysis appear daunting. However, the availability of DNA databases listing the sequence of every potentially expressed protein combined with rapid advances in technologies capable of identifying the proteins that are actually expressed now make proteomics a realistic proposition. Mass spectrometry is one of the essential legs on which current proteomics technology stands.

Quantitative proteomics is the systematic analysis of all proteins expressed by a cell or tissue with respect to their quantity and identity. The proteins expressed in a cell, tissue, biological fluid or portein complex at a given time precisely defines the state of the cell or tissue at that time. The quantitative and qualitative differences between protein profiles of the same cell type in different states can be used to understand the transitions between respective states. Traditionally, proteome analysis was performed using a combination of high resolution gel electrophoresis, in particular two-dimensional gel electrophoresis, to separate proteins and mass spectrometry to identify proteins. This approach is sequential and tedious, but more importantly is fundamentally limited in that biologically important classes of proteins are essentially undetectable. WO99/12040 discloses mass spectrometric detection of polypeptides. Courchesne et al. (Methods in Molecular Biology vol. 112, 1999, p-487-511 discloses identification of proteins by MALOI mass spectrometry. Corthals et al. (Proteome Research: Two-Dimensional Gel Electrophoresis and Identification methods, 1999, p. 1, 17) discloses identification of proteins by mass spectrometry. Pennington et al. (Trends in Cell Biology, vol. 7, no.7, April 1997, p. 168-173) discloses proteome analysis.

Thus, there exists a need for rapid, efficient, and cost effective methods proteome analysis. The present invention satisfies this need and provides related advantages as well.

According to the present invention there is provided a method of identifying a polypeptide as claimed in claim 1.

The invention provides a method for identifying polypeptides. A method is disclosed which includes the steps of simultaneously determining the mass of a subset of parent polypeptides from a population of polypeptides and.the mass of fragments of the subset of parent polypeptides; comparing the determined masses to an annotated polypeptide index; and identifying one or more polypeptides of the annotated polypeptide index having

the determined masses. A method for identifying a polypeptide is disclosed which comprises determining two or more characteristics associated with the polypeptide, or a fragment thereof, one of the characteristics being mass of a fragment of the polypeptide, the fragment mass being determined by mass spectrometry; comparing the characteristics associated with the polypeptide to an annotated polypeptide index; and identifying one or more polypeptides in the annotated polypeptide index having the characteristics. The method can additionally include the step of quantitating the amount of the identified polypeptide in a sample containing the polypeptide.

Various embodiments of the present invention will now be described, by way of example only, and with reference to the accompanying drawings in which:
Figure 1 shows a schematic diagram of a protein identification strategy based on mass spectrometry {MS) and tandem mass spectrometry (MS/MS) measurements.

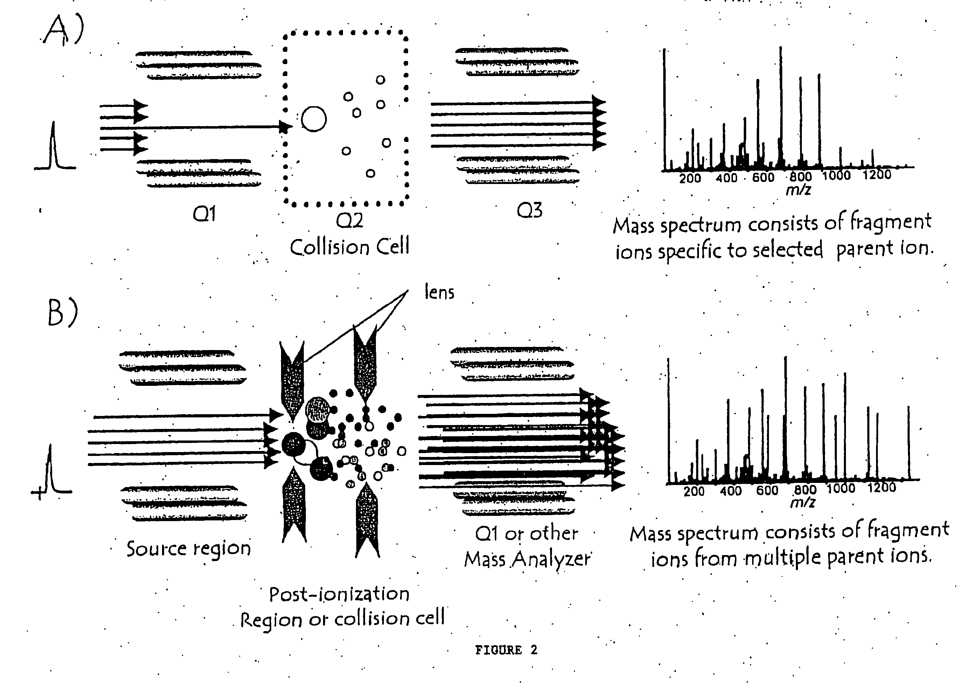

Figure 2 shows two different methods to generate fragment ion selected peptide ions that are diagnostic for the identification of the parent ion. Figure 2A shows the selection of a parent ion (in Q1), which is fragmented in a collision cell (Q2). A mass spectrum of the fragments is determined (in Q3). Figure 2B shows that, instead of selecting a single parent ion, multiple parent ions (indicated in "Source region") are concurrently fragmented in the post-ionization region or collision cell. The fragment ions are then analyzed in a Q1 or other mass analyzer, resulting in a mass spectrum consisting of fragment ions from multiple parent ions.
Figure 3 shows the steps of a method for comparing and quantitating two polypeptide populations using a polypeptide identification index of Annotated Peptide Tags.
Figure 4 shows identification of a polypeptide using mass spectrometry (MS). Figures 4A and 4B show mass spectra of two polypeptides (P1, ASHLGLAR, SEQ ID NO:1; and P2, RPRGFSPR, SEQ ID NO:2) obtained using ESI-TOF. Spectra were acquired at low V_{Nozzle-Skimmer} (10V) (Figure 4A) and high V_{Nozzle-Skimmer} (240V) (Figure 4B). Figure 4C shows a list of 12 and 13 possible polypeptide identifications for Pl (SEQ ID NOS:3-12, 2, 13 and 14, respectively) and P2 (SEQ ID NOS:15-24, 1 and 25 respectively), respectively.
Figure 5 shows chromatographic analysis of a *Saccharomyces cerevisiae* extract. The peptide indicated by the arrow was analyzed by non-fragmentary and fragmentary MS analysis.
Figure 6 shows non fragmentary MS analysis of the peptide indicated by the arrow in Figure 5 (YRPNCPIILVTR; SEQ ID NO:26).
Figure 7 shows fragmentary MS analysis of the peptide indicated by the arrow in Figure 5 (YRPNCPIILVTR; SEQ ID NO:26).
Figure 8 shows chromatographic analysis. Figure 8A shows the base peak chromatogram, Figure 8B shows the selected ion chromatogram at 496.26 m/z [M₁ +3H], and Figure 8C shows the selected ion chromatogram at 586.30 m/z [M₂ +3H].
Figure 9 shows non fragmentary MS analysis.
Figure 10 shows fragmentary MS analysis.

A method for identifying a polypeptide from a population of polypeptides is disclosed by determining characteristics associated with a polypeptide, or a peptide fragment thereof, comparing the determined characteristics to a polypeptide identification index; and identifying one or more polypeptides in the polypeptide identification index having the same characteristics. The methods are applicable to proteome analysis and allow rapid and efficient identification of one or more polypeptides in a complex sample. The methods are based on generating a polypeptide identification index, which is a database of characteristics associated with a polypeptide. The polypeptide identification index can be used for comparison of characteristics determined to be associated with a polypeptide from a sample for identification of the polypeptide. Furthermore, the methods can be applied not only to identify a polypeptide but also to quantitate the amount of specific proteins in the sample.

The methods for identifying a polypeptide are applicable to performing quantitative proteome analysis, or comparisons between polypeptide populations that involve both the identification and quantitation of sample polypeptides. Such a quantitative analysis can be conveniently performed in two separate stages, if desired. As a first step, a reference polypeptide index can be generated representative of the samples to be tested, for example, from a species, cell type or tissue type under investigation, as described herein. The second step is the comparison of characteristics associated with an unknown polypeptide with the reference polypeptide index or indices previously generated. A reference polypeptide index is a database of polypeptide identification codes representing the polypeptides of a particular sample, such as a cell, subcellular fraction, tissue, organ or organism. A polypeptide identification index can be generated that is representative of any number of polypeptides in a sample, including essentially all of the polypeptides potentially expressed in a sample. Accordingly, the disclosed methods advantageously allow the determination of polypeptides in a sample that correlates with or defines a particular physiological state of the sample, for example, a disease state. Moreover, once a polypeptide identification index has been generated, the index can be used repeatedly to identify one or more polypeptides in a sample, for example, a sample from an individual potentially having a disease.

For quantitation of a polypeptide in a sample, a polypeptide is compared to a chemically identical molecule that is isotopically labeled, for example, with ¹³C for ¹²C, deuterium for hydrogen, or ¹⁸O for ¹⁶O. Any number of differential isotopes can be incorporated so long as there is a sufficient difference in mass to be distinguished by MS, as disclosed herein. Because the molecules are chemically identical except for the isotopic difference, the molecules behave physicochemically the same. Furthermore, if desired, more than two samples can be compared if a sufficient number of isotopic labels (for example, d0, d4, d8, d12) are available such that the multiple samples can be compared and distinguished by MS. Quantitation is based on stable isotope dilution. One method to quantitate a sample is to spike a sample with an internal standard that is chemically identical but isotopically different. A standard curve can be generated with dilution of isotope to extrapolate the quantity of molecule in a sample. In such a case, the molecule to be spiked must be identical and therefore the molecules in the sample must be known.

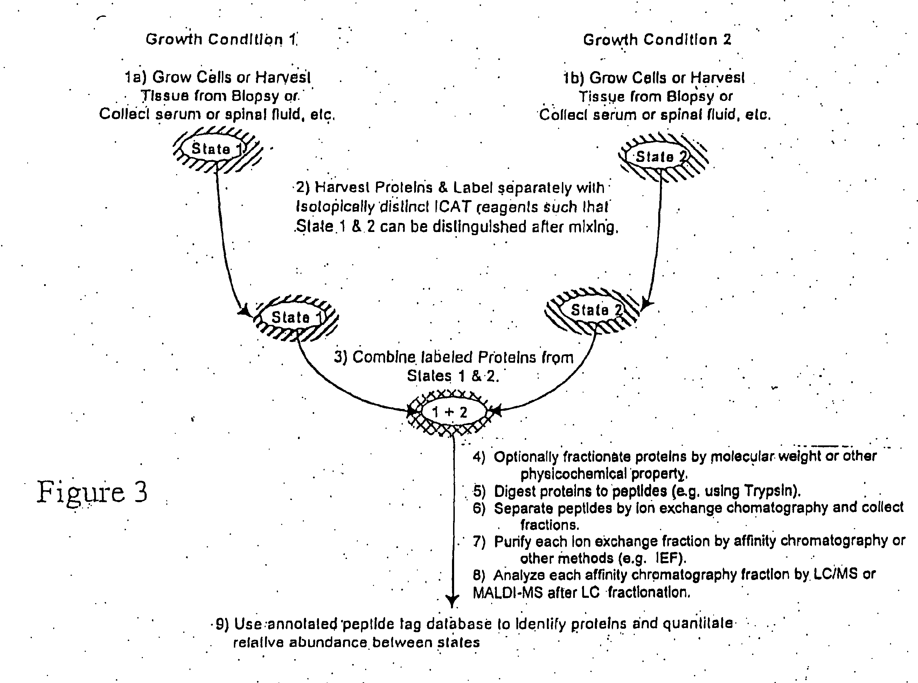

Another convenient method for quantitating polypeptides in a sample is to use a reagent such as ICAT™ (Gygi et al., Nature Biotechnol. 17:994-999 (1999); WO 00/11208). An ICAT™ type reagent, which is described in more detail below, contains an affinity tag, a linker moiety in which one or more stable isotopes can be incorporated, and a reactive group that can covalently couple to an amino acid side chain in a polypeptide such as a cysteine. For quantitation using an ICAT™ type reagent, parallel samples are treated with different isotopic versions of the ICAT™ type reagent. A sample can be labeled and compared to a parallel labeled sample, for example, to normalize to a reference or control sample for quantitation. The use of an ICAT™ type reagent to identify and quantitate polypeptides in a sample is illustrated in Figure 3. Because the peptides labeled with different isotopic versions of the ICAT™ type reagent behave physicochemically the same, the same polypeptides in the two samples will co-purify but still be distinguishable by MS due to the isotopic differences in the ICAT™ type label. Accordingly, the relative amounts of the same polypeptides can be readily compared and quantitated (Gygi et al., *supra*, 1999). Every other scan can be devoted to fragmenting and then recording sequence information about an eluting peptide (MS/MS spectrum). The parent polypeptide that this peptide originated from can be identified by searching a sequence database with the recorded MS/MS spectrum. The procedure thus provides the relative quantitation and identification of the components of protein mixtures in a single analysis. Such a comparison can be useful for quantitating the expression levels of polypeptides relative to a reference sample, for example, comparing expression levels in a sample from an individual having a disease or suspected of having a disease to a sample from a healthy individual or for forensic purposes.

In addition to being useful for quantitation of polypeptides, an ICAT™ type reagent also functions as a constraint on the complexity of the system, that is, only polypeptides or fragments thereof containing the amino acid reactive with the ICAT™ type reagent will be labeled and characterized if the polypeptides are affinity isolated or compared side-by-side with a differentially isotopically labeled sample (Gygi et al., supra, 1999). Accordingly, the use of an ICAT™ type reagent can provide a reduction in complexity of the sample. Furthermore, the ability of a polypeptide or fragment thereof to be labeled with an ICAT™ type reagent, that is, whether the peptide contains the reactive amino acid, is a characteristic associated with the polypeptide useful for identifying the polypeptide in combination with additional characteristics.

An additional advantage of the use of an ICAT™ type reagent is that the identity of polypeptides in a sample need not be known prior to analysis. As described above, isotopic dilution, where an internal standard is spiked into a sample, requires that a chemically identical molecule that is differentially isotopically labeled be spiked into the sample and, therefore, requires that a polypetpide or fragment thereof to be quantitated is known so that a chemically identical isotopically labeled molecule be added. With an ICAT™ type reagent, no prior knowledge of the exact polypeptides or fragments need be known. Furthermore, there is no need to synthesize a variety of isotopically labeled molecules for characterizing a variety of polypeptides in a sample.

In addition to using a labeling reagent such as an ICAT™ type reagent that incorporates an affinity label, other labeling reagents can be used to differentially isotopically label two different samples containing polypeptides. For example, two chemically identical reagents containing different isotopes can be used to covalently modify two polypeptide samples, where the reagents do not contain an affinity tag. Accordingly, instead of using an affinity isolation step associated with an ICAT™ type tag, other isolation steps, if desired, can be used. Nevertheless, the differentially isotopically labeled polypeptide samples can be compared for quantitative analysis. For example, methylation of polypeptides via esterification with methanol containing d0 (no deuterium) versus d3 (three deuteriums) can be used to differentially isotopically label two polypeptide samples. Similarly, any of the well known methods for modifying side chain amino acids in polypeptides can analogously be used with differentially labeled isotopes such as deuterium for hydrogen, C¹³ for C¹², O¹⁸ for O¹⁶ (see, for example, Glazer et al., Laboratory Techniques in Biochemistry and Molecular Biology: Chemical Modification of Proteins, Chapter 3, pp. 68-120, Elsevier Biomedical Press, New York (1975); Pierce Catalog (1994), Pierce, Rockford IL). Any number of the differential isotopes can be incorporated so long as parallel labeled polypeptides contain a sufficient mass distinction to be detected by MS. In addition to chemical modification of a polypeptide, as described above, two polypeptide samples can be digested with a protease such as trypsin or the like in the presence of O¹⁶- versus O¹⁸-labeled H₂O. Since the protease cleavage reaction results in the addition of water to the cleaved peptides, cleavage in the presence of isotopically differentially labeled H₂O can be used to incorporate differential labels into separate polypeptide samples. It is understood that any method useful for incorporating an isotopic label to differentially label two polypeptide samples can be used in the disclosed methods, particularly for quantitative methods, so long as the samples to be compared are treated in a chemically similar fashion such that the resulting labeled polypeptides essentially differ only by the differential isotopic label.

Still another method to quantitate a sample is to incubate a sample under conditions that allow metabolic incorporation of isotopes into two samples for comparison by incubating a sample in the presence of an isotope or incubating in media that results in depletion of a naturally occurring isotope (see, for example, Oda et al., Proc. Natl. Acad. Sci. USA 96:6591-6596 (1999)). Such a method is particularly useful for a sample that is conveniently cultured, for example, a microbial sample or a primary culture of cells obtained from an individual. Accordingly, both *in vitro* and in vivo methods can be used to differentially isotopically label two samples for comparison and/or quantitation.

The disclosed methods are based on determining characteristics of a polypeptide that allow identification of the polypeptide based on the determined physicochemical characteristics. The collection of physicochemical characteristics that can function to identify a polypeptide is essentially a "bar code" for the polypeptide, that is, a collection of characteristics sufficient to uniquely identify a polypeptide based on correlating the characteristics with a reference database that functions as a polypeptide identification index. The methods are particularly advantageous for rapid and efficient analysis of complex samples containing many different polypeptides, which would be time consuming and inefficient using other methods. The methods can thus be applied to analyze complex samples containing numerous different polypeptides and are particularly useful in proteomics applications. Accordingly, the methods can be advantageously used to identify polypeptides of the proteome. Since the proteome reflects polypeptide expression and post-translational modifications correlated with the metabolic state of the cell, the methods can also be used in diagnostic applications to determine normal or aberrant polypeptide expression associated with a disease. Accordingly, the disclosed methods can be used in clinical applications to diagnose a disease or condition.

The disclosed methods advantageously use constraining parameters that allow the identification of a polypeptide from a complex mixture of different polypeptides. The constraints can be used to simplify the identification of polypeptides. A constraint can be, for example, the inclusion of one or more additional characteristics associated with a polypeptide, the identification of a subset of polypeptides from a complex mixture, or any type of constraint that can be used to simplify the analysis of a complex mixture of polypeptides. The disclosed methods thus provide more efficient identification of polypeptides in a complex mixture, including large numbers of polypeptides, which is particularly useful for proteome analysis.

The generation and use of a polypeptide identification index provide several advantages. First, the methods can be used with selective isolation of polypeptide fragments containing specific structural features, which can be exploited by tagging with specific chemical reagents. The affinity selection of "tagged" fragments simplifies the polypeptide mixture, rendering it compatible with highly denaturing/solubilizing conditions that can be used for protein isolation and handling. The selective isolation of fragments also constrains database searching. For example, selective cysteine tagging, as disclosed herein, reduces the complexity of the peptide mixture by approximately 10-fold.

A second advantage of the disclosed methods is that they can be readily used in a variety of laboratory settings. For example, mass measurements are absolute and chromatographic parameters can be easily standardized. Therefore, a polypeptide identification index determined by the disclosed methods is easily transferable between laboratories, and data generated by different laboratories can be easily compared with a polypeptide identification index generated under similar conditions. This advantage can be further exploited by making the method accessible via a network, for example, through the construction of a Web-based search tool. A third advantage is that the methods can be performed with a single stage mass analysis, which is fast, simple and sensitive. A fourth advantage is that the methods can be used to accurately measure the ratio of each polypeptide present in a complex polypeptide sample, provided that the samples have been modified with a stable isotope label. Finally, the methods have an essentially unlimited sample capacity, assuring the possibility of analyzing polypeptides of very low abundance, and have a high peak capacity, allowing for the analysis of very complex samples.

As disclosed herein, in addition to isolating individual parent ions prior to fragmentation, multiple ions can be fragmented in parallel without single ion selection (see Figure 2). Accordingly, a key advantage of such a method is that the parameters can be easily determined in parallel for multiple polypeptides rather than separately for each peptide as is the case in protein identification by MS/MS.

A polypeptide identification index may be generated by determining characteristics associated with a polypeptide, in particular, fragment ion mass measurements by MS/MS generated with or without parent ion selection (Figure 2) and optionally including chromatographic steps. These mass determinations are not required to be at high accuracy. The accurate mass can be calculated, if desired, and compiled into an index with other characteristics associated with a particular polypeptide. A sufficient number of characteristics are determined to allow identification of a polypeptide in the index. The methods can optionally and advantageously be used with quantitation to provide additional information on the physiological state of a sample. However, in the case of simpler systems, for example, microbial or viral genomes or specimens from an individual containing a smaller number of polypeptides such as spinal fluid, the complexity of polypeptides in a sample can be sufficiently small enough that qualitative analysis of the polypeptides in a sample is sufficient for particular applications. As such, if a qualitative determination of the expression of a polypeptide in a sample is sufficient to correlate with a particular condition, for example, a disease condition, then the disclosed methods can be applied to a qualitative identification of a polypeptide in a sample.

An exemplary polypeptide identification index or database is described in Example IV. The growing field of mass spectrometrically based proteomics requires the incorporation of new methods of rapidly identifying proteins from whole cell state interrogations. Towards this end, sample simplification methods have been successfully employed, including cysteine-containing peptide purification (Gygi et al., Nat. Biotechnol. 17:994-999 (1999)). Using the constraints that naturally fall from this, the potential number of peptide candidates in a mixture can be reduced to the point that high mass accuracy (- 0.1-1 ppm) can conclusively identify a peptide and its parent protein (Goodlett et al., Anal Chem. 72:1112-1118 (2000)). Without the use of a cysteine-constraint, it has been shown that high mass accuracy of a peptide and few fragment ions from that peptide can achieve the same result, even in the presence of co-eluting peptides (Masselon et al., Anal Chem. 73:1918-1924 (2000)). As disclosed herein, a combination of these concepts using a TOF rather than an FT-ICR mass spectrometer was tested for an extract of *Saccharomyces cerevisiae* (see Example IV). This was done by a combination of cysteine-constraint to reduce database complexity and in-source CID to generate fragment ions. The method was demonstrated to be effective even when peptides co-elute. This process allows shotgun sequencing to reflect sequencing of co-eluting peptides.

For example, as shown in Figure 2, a method can be performed in the absence of single ion selection or in the absence of ion selection in a source region. The regions designated, for example, as Q₁, Q₂, Q₃ and the like, refers to quadrupoles. These are physical means to separate a selected ion based on m/z. However, it is understood that any appropriate methods suitable for separating selected iocs, in addition to the use of quadrupoles, can be used in the methods.

As used herein, the term "characteristic" when used in reference to a polypeptide refers to a physicochemical property of a polypeptide. Physicochemical properties include physicochemical properties of a parent polypeptide such as molecular mass, amino acid composition, pI and the like, as well as physicochemical properties of a fragment of a polypeptide, including fragment ions, which can be correlated with a polypeptide and are thus considered to be characteristics associated with a parent polypeptide. Physicochemical properties of a polypeptide also include measurable behaviors of a polypeptide that result from its particular physicochemical properties. For example, physicochemical properties include the order of elution on specific chromatographic media under defined conditions, and the position to which a polypeptide migrates in a polyacrylamide gel under defined conditions. The characteristics can be determined empirically or can be predicted based on known information about the polypeptide, for example, sequence information.

As used herein, the term "characteristic associated with a polypeptide" refers to a physicochemical property of a polypeptide and/or any fragment of the polypeptide. As such, a characteristic associated with a polypeptide include specific characteristics of a parent polypeptide as well as characteristics of a fragment of the parent polypeptide which, because the fragment can be related to the polypeptide, are considered to be characteristics associated with the parent polypeptide. Such characteristics can be used to identify a polypeptide, for example, by comparison with a polypeptide identification index.

As used herein, the term "polypeptide" refers to a peptide or polypeptide of two or more amino acids. A polypeptide can also be modified by naturally occurring modifications such as post-translational modifications, including phosphorylation, lipidation, prenylation, sulfation, hydroxylation, acetylation, addition of carbohydrate, addition of prosthetic groups or cofactors, formation of disulfide bonds, proteolysis, assembly into macromolecular complexes, and the like.

A modification of a polypeptide, particularly ligand polypeptides, can also include non-naturally occurring derivatives, analogues and functional mimetics thereof generated by chemical synthesis, provided that such polypeptide modification displays a similar functional activity compared to the parent polypeptide. For example, derivatives can include chemical modifications of the polypeptide such as alkylation, acylation, carbamylation, iodination, or any modification that derivatizes the polypeptide. Such derivatized molecules include, for example, those molecules in which free amino groups have been derivatized to form amine hydrochlorides, p-toluene sulfonyl groups, carbobenzoxy groups, t-butyloxycarbonyl groups, chloroacetyl groups or formyl groups. Free carboxyl groups can be derivatized to form salts, methyl and ethyl esters or other types of esters or hydrazides. Free hydroxyl groups can be derivatized to form O-acyl or O-alkyl derivatives. The imidazole nitrogen of histidine can be derivatized to form N-im-benzylhistidine. Also included as derivatives or analogues are those polypeptides which contain one or more naturally occurring amino acid derivatives of the twenty standard amino acids, for example, 4-hydroxyproline, 5-hydroxylysine, 3-methylhistidine, homoserine, ornithine or carboxyglutamate, and can include amino acids that are not linked by peptide bonds.

A particularly useful polypeptide derivative includes modification of sulfhydryl groups, for example, the modification of sulfhydryl groups to attach affinity reagents such as an ICAT™ type reagent. A particularly useful modification of a polypeptide includes modification of polypeptides in a sample with a moiety having a stable isotope. For example, two different polypeptide samples can be separately labeled with moieties that are isotopically distinct, and such differentially labeled samples can be compared. Modification of polypeptides with stable isotopes is particularly useful for quantitating the relative amount of individual polypeptides in a sample.

As used herein, a "fragment" refers to any truncated form, either carboxy-terminal, amino-terminal, or both, of a parent polypeptide. Accordingly, a deletion of a single amino acid from the carboxy- or amino-terminus is considered a fragment of a parent polypeptide. A fragment generally refers to a deletion of amino acids at the N- and/or C-terminus but also includes modifications where a side chain is removed but the peptide bond remains. A fragment includes a truncated polypeptide that is generated, for example, by polypeptide cleavage using a chemical reagent, enzyme, or energy input. A fragment can result from a sequence-specific or sequence independent cleavage event. Examples of reagents commonly used for cleaving polypeptides include enzymes, for example, proteases, such as thrombin, trypsin, chymotrypsin and the like, and chemicals, such as cyanogen bromide, acid, base, and o-iodobenzoic acid, as disclosed herein. A fragment can also be generated by a mass spectrometry method. Furthermore, a fragment can also result from multiple cleavage events such that a truncated polypeptide resulting from one cleavage event can be further truncated by additional cleavage events.

As used herein, the term "polypeptide identification index" refers to a collection of characteristics associated with a polypeptide sufficient to identify and distinguish other polypeptides in the index. A polypeptide identification index is therefore a collection of polypeptide identification codes for identifying a polypeptide based on characteristics of the polypeptide or a fragment thereof. A polypeptide identification index can be based on deduced characteristics associated with a polypeptide, for example, characteristics predicted based on sequence information such as genomic sequence, cDNA sequence, or EST databases. A polypeptide identification index can also be based on empirically determined characteristics, or a combination of deduced and empirically determined characteristics. An "annotated polypeptide (AP) index" refers to a polypeptide identification index comprising at least one empirically determined characteristic for each of the polypeptides in the index, which can be determined, for example, by the methods disclosed herein. If desired, an AP index can be based on entirely empirically determined characteristics or a combination of deduced and empirically determined characteristics. The use of an annotated polypeptide index is particularly useful for identifying polypeptides modified by post-translational modifications, which can have characteristics unpredictable based on deduction from a sequence database alone.

A "polypeptide identification subindex" refers to a subset of a polypeptide identification index that contains less than all of the polypeptide identification codes of the polypeptide identification index. A subindex can contain, for example, five polypeptide identification codes from a polypeptide identification index of ten polypeptide identification codes, which is a subset of the entire index. Identification of a subindex can be useful, for example, for reducing the complexity of a search of a polypeptide identification index, similar to the reduction in complexity that can be applied to a polypeptide sample by the fractionation methods disclosed herein. Accordingly, a search of a subindex can be advantageous in requiring less computational time than required to search an entire index.

As used herein, the term "identification code" refers to a set of characteristics associated with a polypeptide that is sufficient to determine the identity of the polypeptide and distinguish the polypeptide from other polypeptides in a polypeptide identification index. An identification code is essentially an annotated peptide tag, or "bar code," that can be used to identify a polypeptide.

The invention provides a method for identifying a polypeptide. The method includes the steps of determining two or more characteristics associated with a polypeptide or fragment thereof, one of the characteristics being the mass of a fragment of the polypeptide, wherein the fragment mass is determined by mass spectrometry; comparing the characteristics associated with the polypeptide to a polypeptide identification index such as an annotated polypeptide index; and identifying one or more polypeptides in the polypeptide identification index having the determined characteristics. The fragment can be determined at an accuracy in ppm of greater than 1 part per million (ppm) or at even lower accuracy (higher ppm). The method can further include determining one or more additional characteristics associated with the polypeptide and comparing the characteristics determined in each of the steps to the polypeptide identification index. Optionally, the steps of determining one or more additional characteristics associated with the polypeptide and comparing the characteristics determined in each step to the polypeptide identification index can be repeated one or more times, wherein a set of characteristics is determined that identifies a single polypeptide in the polypeptide identification index. The above method, as well as other methods, can further include quantitating the amount of polypeptide in a sample. Furthermore, the methods can be used to measure the relative abundance in two or more different populations of polypeptides, that is, polypeptide mixtures, for example, populations of polypeptides in different samples.

The methods for identifying a polypeptide include determining characteristics associated with the polypeptide, or a fragment of the polypeptide. Characteristics associated with a polypeptide that are useful for identifying a polypeptide are those characteristics that can be reproducibly determined. Physicochemical properties of a polypeptide or fragment include, for example, atomic mass, amino acid composition, partial amino acid sequence, apparent molecular weight, pI, and order of elution on specific chromatographic media under defined conditions. Such characteristics determined to be associated with a polypeptide are used for the identification of the polypeptide. Methods for determining characteristics associated with a polypeptide are described in more detail below.

One of the characteristics particularly useful in the disclosed methods is the mass of a polypeptide or a fragment or fragments thereof. A fragment of a polypeptide can be generated prior to or during the process of mass determination by mass spectrometry. A polypeptide fragment mass can therefore be the mass of a fragment of a polypeptide generated during polypeptide sample preparation, or can be the mass of fragment generated by a polypeptide cleavage that occurred during mass spectrometry.

In the disclosed methods, the mass of a polypeptide fragment is determined by mass spectrometry, and can advantageously be determined in the absence of ion selection for producing fragment ions. The disclosed methods allow the identification of a polypeptide without the need for sequencing the polypeptide or fragment thereof. A polypeptide fragment mass can be determined using a variety of mass spectrometry methods known in the art, as described herein.

A variety of mass spectrometry systems can be employed in the disclosed methods for identifying a polypeptide. Mass analyzers with high mass accuracy, high sensitivity and high resolution include, but are not limited to, matrix-assisted laser desorption time-of-flight (MALDI-TOF) mass spectrometers, ESI-TOF mass spectrometers and Fourier transform ion cyclotron mass analyzers (FT-ICR-MS). Other modes of MS include an electrospray process with MS and ion trap. In ion trap MS, fragments are ionized by electrospray or MALDI and then put into an ion trap. Trapped ions can then be separately analyzed by MS upon selective release from the ion trap. Fragments can also be generated in the ion trap and analyzed. The ICAT™ type reagent labeled polypeptides that can be used in the disclosed methods can be analyzed, for example, by single stage mass spectrometry with a MALDI-TOF or ESI-TOF system. Mass spectrometry methods for proteomics applications have been described (see Aebersold and Goodlett, Chem. Rev. 101:269-295 (2001)). If desired, MS methods can be modified to allow detection of affinity tagged peptides, for example, using ICAT™ or IDEnT type reagents, as described herein.

If desired, different MS analysis can be applied for generating a polypeptide identification index than for determining characteristics of an unknown polypeptide. For example, LC-MS/MS can be used for collecting data for the identification index and LC-ESI-TOF can be used for measurement of characteristics of an unknown polypeptide. It is understood that any MS methods and any combination of MS methods can be used so long as the samples are treated in a substantially similar manner and so long as the MS methods are compatible for comparison of masses determined by the different methods.

The disclosed methods can involve a polypeptide separation step followed by a mass analysis step. Polypeptide separation and mass analysis steps can be performed independently or can be coupled in an "on line" analysis method. Various modes of polypeptide separation techniques can be coupled to a mass analyser. For example, polypeptides can be separated by chromatography using microcapillary HPLC, by solid phase extraction-capillary electrophoresis systems that can be coupled to a mass analyzer, or by gel electrophoresis methods. A specific example of a coupled polypeptide separation and mass analysis method is micro-capillary HPLC coupled to an ESI-MS/MS system that is applied with dynamic exclusion on an ion trap MS.

Different types of mass spectrometry can be used for different applications of the disclosed methods. For certain applications, such as mass determination of a polypeptide fragment for generating a polypeptide identification index, a method that provides high accuracy, such as an accuracy of less than 1 part per million. However, the disclosed methods are advantageous in that MS of lower accuracy, that is higher ppm resolution, can be conveniently used without the need for more expensive instrumentation required for higher accuracy determinations. For applications that involve high throughput analysis of a population of polypeptides, a lower accuracy mass determination can be sufficient. Lower accuracy mass determinations generally provide higher sample throughput because less time is required to make a mass determination.

The disclosed methods involving mass determinations can be conveniently performed at lower accuracy. For example, high mass accuracy instruments such as FTMS or FTICR MS can be used to determine accuracy at 0.2 ppm (Goodlett et al. et al., Anal. Chem. 72:1112-1118 (2000); Masselon et al., Anal. Chem. 72:1918-1924 (2000)). The use of very high mass accuracy such as 0.1 ppm acts as a constraint. However, the disclosed method are advantageous in that several characteristics associated with a polypeptide can be determined. When combined with additional characteristics, the masses can be determined at lower accuracy, that is higher ppm. Determination of mass at lower accuracy allows the use of less expensive MS instruments which are more widely available than FTMS. The mass determinations can be determined at an accuracy in ppm of 1 part per million (ppm) or greater than 1 ppm, and can be determined at an accuracy in ppm of 2.5 ppm or greater, of about 5 ppm or greater, about 10 ppm or greater, about 50 ppm or greater, about 100 ppm or greater, about 200 ppm or greater, about 500 ppm or greater, or even about 1000 ppm or greater, sequentially each of which requires less accuracy of the MS instrument. The disclosed methods advantageously allow the use of lower accuracy MS analysis in combination with other physicochemical characteristics, as disclosed herein, to identify a polypeptide in a sample. The accuracy of the MS measurement for a particular application can be readily determined by one skilled in the art, for example, depending on the complexity of the sample and/or index to be used.

The disclosed methods for identifying a polypeptide can involve determining the mass of a polypeptide fragment at an accuracy of greater than 1 part per million. Therefore, the method does not require a MS method having high accuracy. Accordingly, a lower-cost MS system can be employed in the methods of identifiying a polypeptide. The adaptation of any mass spectrometer to a high throughput format, such as 96-well plate or 384 spot plate format, or to an autoinjection system that allows unattended operation, is advantageous for increasing sample throughput.

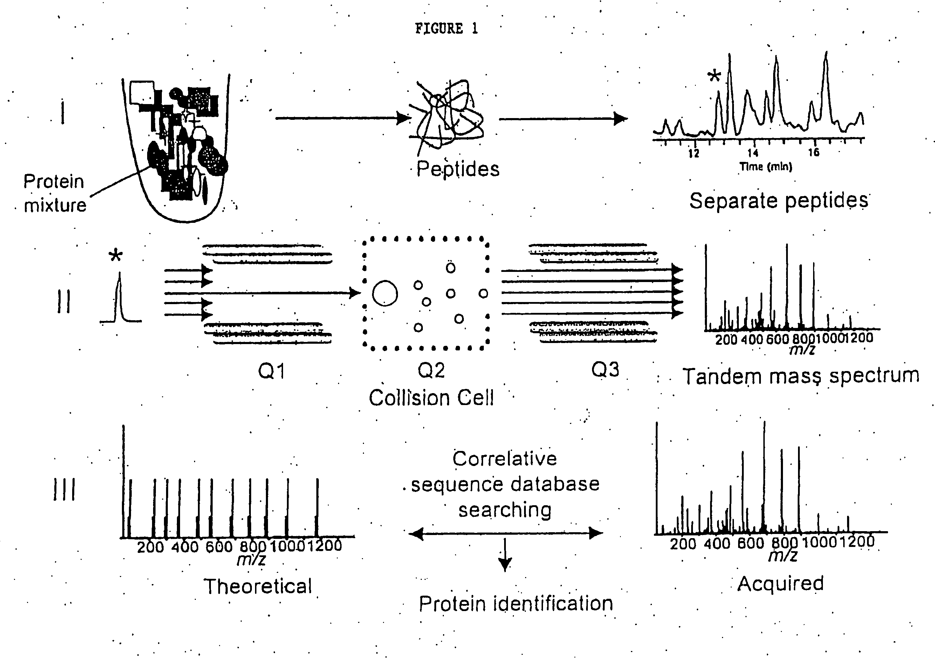

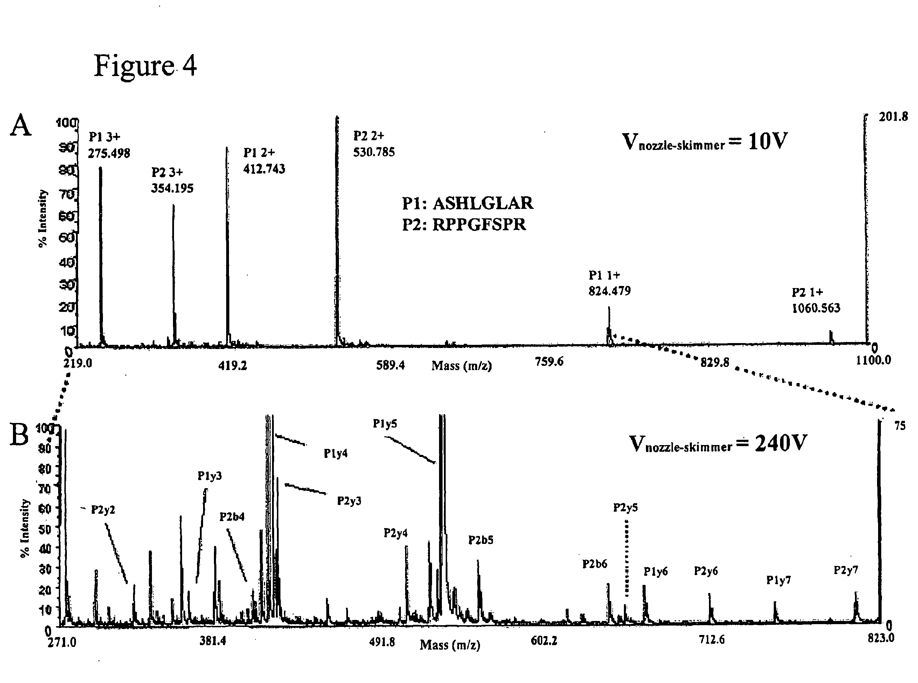

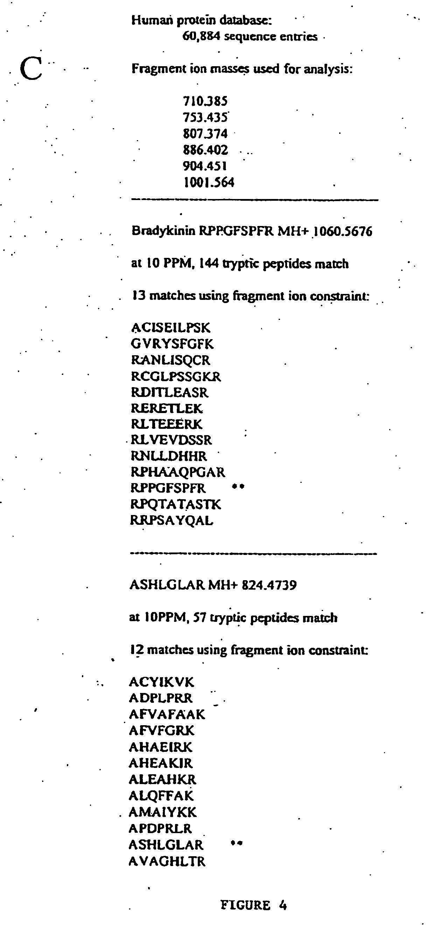

The mass of a polypeptide or fragment thereof can be determined in the absence of ion selection for producing fragment ions. An overview of the strategy of a protein identification method is shown in Figure 1. Polypeptides are optionally fractionated, for example, using polyacrylamide gel electrophoresis, and the polypeptides can further be fragmented into peptides. The peptides can further be optionally fractionated by chromatography. A chromatographic fraction, or bin (indicated by "*" in Figure 1), is subjected to MS. Traditionally, an ion or dominant ions are selected in a collision cell for collision-induced dissociation (CID). Selection of a single ion is depicted in Q1 of Figure 1. An ion is selected and then fragmented, as shown in Q3 of Figure 1. In the absence of ion selection, instead of a single ion being selected, no selection of ions is applied but, rather, all of the ions are fragmented, leading to many peptide fragments. The peptide fragments are deconvoluted to determine which correspond to a particular parent polypeptide, and such information on the mass of a fragment of a polypeptide is a characteristic associated with the polypeptide (see Figure 4). As shown in the bottom of Figure 1, the fragment masses can be combined with any number of additional characteristics and compared to a protein identification index, for example, a sequence database or an annotated polypeptide index, and the polypeptide is identified based on those determined characteristics.

A set of determined characteristics associated with a polypeptide are compared to the characteristics associated with a polypeptide in a polypeptide identification index. A polypeptide identification index is a collection of characteristics associated with individual polypeptides that uniquely identify and distinguish the polypeptides from other polypeptides annotated in the index. By comparing the set of determined characteristics associated with a polypeptide to a polypeptide identification index, one or more polypeptides in the polypeptide identification index that share the same characteristics can be identified. If more than one polypeptide is determined to have the same characteristics, additional constraints can be included, for example, the determination of one or more additional characteristics. A polypeptide identification index can be based on deduced characteristics of a polypeptide, for example, one or more characteristics deduced from genetic sequence databases, or can be determined empirically, as with the annotated peptide tag index described herein.

One exemplary method of generating an annotated peptide index is to: harvest proteins; label proteins with an isotope coded affinity tag (ICAT™) type reagent; fractionate proteins by molecular weight; digest proteins to peptides (e.g. using trypsin); separate peptides by ion exchange; purify each ion exchange fraction by affinity chromatography; analyze each affinity chromatography fraction by LC/MS/MS (or CE/MS/MS); identify all expressed proteins via database search of individual MS/MS peptide spectra; generate a database of annotated peptide tags that constitute a unique barcode for an individual peptide based on measured physicochemical properties and thus the parent protein of that peptide. It is understood that the above-described method, combinations of these steps, modifications thereof, or any methods suitable to allow the determination of characteristics associated with a polypeptide can be used to generate a polypeptide identification index containing at least one empirically determined characteristic, as described herein.

The disclosed methods can further include determining one or more additional characteristics associated with the polypeptide for comparison with a polypeptide identification index. The process of determining one or more additional characteristics associated with a polypeptide followed by comparing with a polypeptide identification index can be repeated until a single polypeptide is uniquely identified from the polypeptide identification index. Accordingly, if additional constraints are applicable, they can be included to identify a polypeptide by comparison to a polypeptide identification index (see Figure 4C).

The number of characteristics sufficient to identify of a polypeptide can be readily determined by one skilled in the art by comparing the determined set of characteristics with the polypeptide identification index. The identification of a single polypeptide in a polypeptide identification index refers to determining a set of characteristics that are sufficient to distinguish the polypeptide from another polypeptide in the polypeptide identification index. For example, if two determined characteristics match a single polypeptide in a polypeptide identification index, then the two characteristics are sufficient to identify a single polypeptide. Similarly, for a different polypeptide, three determined characteristics can be required to uniquely identify a polypeptide in the index. Accordingly, based on the characteristics determined for a polypeptide, a comparison is made to a polypeptide identification index. If a single polypeptide is identified, then a sufficient number of characteristics have been determined. If more than one polypeptide is identified, then one or more additional characteristics can be determined until a single polypeptide uniquely matches the determined characteristics, thereby allowing identification of the polypeptide. Therefore, one skilled in the art can readily determine if a sufficient number of characteristics, based on comparison to a particular polypeptide identification index, have been determined for a polypeptide to allow identification of a unique polypeptide in the polypeptide identification index.

The disclosed methods are advantageously based on the inclusion of selected constraints that allow more efficient identification of a polypeptide, particularly in complex samples containing numerous different polypeptides. The methods can also be advantageously used to identify multiple polypeptides simultaneously from a complex sample. Accordingly, rather than determining a large number of characteristics associated with different polypeptides, the methods can be performed in an iterative manner, if desired, with the inclusion of additional constraints as needed to identify a single polypeptide in a polypeptide identification index.

For example, polypeptides that are homologous generally have segments of high sequence identity. Such polypeptides can arise, for example, from polypeptides having similar function, splice variants of the same nucleic acid, and the like. Polypeptides having segments of high sequence identity can have in common several physicochemical characteristics, particularly in association with homologous fragments of the polypeptide. Polypeptides sharing a high degree of similarity can therefore have a similar or identical set of associated characteristics. For such similar polypeptides, a given set of characteristics sufficient to distinguish two dissimilar polypeptides can be insufficient for the identification of a single polypeptide in a polypeptide identification index when the polypeptides have regions of similarity. In such a case, one or more additional characteristics associated with the polypeptide can be determined, and the determination of additional characteristics can be repeated until the subject polypeptide can be distinguished from each other polypeptide in a polypeptide identification index. The methods of determining a set of characteristics associated with a polypeptide, comparing with a polypeptide identification index, and determining additional characteristics until a single polypeptide in a polypeptide identification index is identified can be applied to one or more polypeptides.

Thus, additional constraints, as needed to identify a polypeptide, can be considered. For example, if more than one polypeptide in a polypeptide identification index has a given set of characteristics, the identification of selected polypeptides of the polypeptide identification index, that is, a subset of polypeptides in the index or a subindex of the index, functions essentially as a constraint. Accordingly, a subsequent comparison to the polypeptide identification index can be made to the subindex, which can reduce the calculation time and provide a more efficient comparison, if desired. An additional constraint can then be considered, for example, an additional characteristic, and compared to the subindex, which can result in a reduction in the number of polypeptides having all of the determined characteristics. Such steps can be optionally repeated until a single polypeptide in the polypeptide identification index is identified. Such an approach is advantageous when determining the identity of multiple polypeptides simultaneously because only those characteristics sufficient to identify a polypeptide need be determined. The methods can thus readily accommodate the determination of the identity of a variety of polypeptides and the complexities associated with proteomics analysis without wasting resources on unnecessary data acquisition.

The disclosed methods for generating a polypeptide identification index involve determining, for two or more polypeptides, a set of characteristics that can be used to identify the polypeptide. A set of characteristics that uniquely identify a polypeptide in a polypeptide identification index define a polypeptide identification code or "bar code" for the polypeptide. A polypeptide identification index can contain a variety of characteristics associated with an indexed polypeptide. Polypeptide characteristics contained in a polypeptide identification index can include polypeptide mass, amino acid composition, partial amino acid composition, for example, the presence of a particular amino acid, pI, order of elution on specific chromatographic media, and one or more polypeptide fragment masses. A polypeptide identification index can additionally include amino acid sequence, references to related polypeptides, database entries or literature, as well as other information relevant to the identification of a polypeptide. The user will know what types of information are useful for a polypeptide index and can include any physicochemical property or information relating to a polypeptide. A polypeptide identification index containing a large number of identification codes for a variety of polypeptides is particularly useful for identifying polypeptides in complex samples.

The disclosed methods directed to identifying a polypeptide are based on comparing characteristics determined for a polypeptide with a polypeptide identification index. A polypeptide identification index can be a commercially or publicly available database such as GenBank (www.ncbi.nlm.nih.gov/GenBank), in which one or more characteristics of a polypeptide are predicted, for example, amino acid composition, mass of a polypeptide or fragment thereof, and the like. In addition, a polypeptide identification index can be based on empirically determined characteristics determined by the methods described herein. A polypeptide identification index can be, for example, empirically determined or a combination of predicted and empirically determined characteristics, for example, like the annotated polypeptide (AP) index disclosed herein, also referred to as an annotated peptide tag (APT) index.

A set of empirically determined characteristics associated with a polypeptide can be determined experimentally using a variety of methods. An exemplary method for polypeptide identification and/or determining characteristics for generating a polypeptide identification index is shown in Figure 1 and described below. The method is useful for defining a polypeptide identification code because the method involves a series of steps, which allow the determination of characteristics associated with a polypeptide, the final step being mass determination of a polypeptide or fragment. The method can include:(i) polypeptide sample preparation; (ii) polypeptide tagging; (iii) optional polypeptide fractionation; (iv) polypeptide fragmentation.; (v) polypeptide fragment separation; (vi) affinity isolation of tagged polypeptide fragments; (vii) high resolution polypeptide fragment separation; (viii) database searching; and (ix) polypeptide identification index construction (see Example I).

For polypeptide sample preparation, polypeptide samples for which quantitative proteome analysis is to be performed are isolated from the respective sources using standard protocols for maintaining the solubility of the polypeptides. Polypeptide samples and preparation of polypeptide samples are discussed in more detail below.

For polypeptide tagging, the polypeptides in the sample can be denatured, optionally reduced, and a chemically reactive group of the polypeptides can be covalently derivatized with a chemical modification reagent. An exemplary reactive group is a sulfhydryl group that represents a side chain of a reduced cysteine residue, which can be derivatized by a reagent such as ICAT™ (Gygi et al., Nature Biotechnol. 17:994-999 (1999)) or IDEnT reagent (Goodlett et al., Anal. Chem. 72:1112-1118 (2000)). Other useful reactive groups include amino or carboxyl groups of polypeptides or specific post-translational modifications, including phosphate, carbohydrate or lipid. Any chemical reaction with specificity for a chemical group in the polypeptide can be applied in this step. The ICAT™ type reagent and IDEnT reagents and methods of use are described in more detail below.

For optional polypeptide fractionation, the mixture of tagged polypeptides can be fractionated using any polypeptide separation procedure. A fractionation procedure useful in the disclosed methods is reproducible, allows polypeptides to remain soluble, and has a high sample and peak capacity. Any optional fractionation technique can be performed to enrich for low abundance proteins and/or to reduce the complexity of the mixture, and the relative quantities can be maintained. Exemplary fractionation methods include, for example, sodium dodecyl sulfate-polyacrylamide gel electrophoresis (SDS-PAGE), chromatographic methods such as size exclusion, ion exchange, hydrophobic, and the like, as disclosed herein. Polypeptide fractionation methods are described in more detail below.

For polypeptide fragmentation, the polypeptides in the sample mixture, or the polypeptides contained in each fraction if optional sample fractionation is employed, can be subjected to sequence specific cleavage, such as cleavage by trypsin. The use of sequence specific cleavage can be particularly useful because the termini of peptides cleaved by a sequence specific method can act as a constraint. However, it is understood that the cleavage method used to generate fragments need not be sequence specific, if desired. Methods useful for cleaving polypeptides in a sequence specific manner are described in more detail below.

For polypeptide fragment separation, the resulting polypeptide fragment mixtures can optionally be subjected to a first dimension peptide separation. Separation methods having a high sample capacity, at least moderate resolving power and highly reproducible separation patterns are useful in this step. Examples of first dimension separation methods include anion and cation ion exchange chromatographies. These and other chromatographic methods are described in more detail below. Although polypeptide fragment separation can optionally be performed, the methods can be advantageously used such that the characteristics of peptide fragments are measured in "bulk," that is, the methods do not require peptide fragment purification to homogeneity.

For affinity isolation of tagged polypeptide fragments, polypeptide fragments can be isolated from each chromatographic fraction using an affinity reagent that binds to the polypeptide tag. For example, polypeptide fragments tagged with the ICAT™ reagent exemplified herein can be isolated using avidin or streptavidin affinity chromatography. An example of a useful affinity medium for isolation of ICAT™ labeled polypeptide fragments is monomeric avidin immobilized on polymer beads. If ICAT™ type reagents with affinity tags different from biotin are used, corresponding affinity media that binds the affinity tag is used. As disclosed herein, affinity isolation separation can be performed in parallel, for example, using a microtiter plate with affinity beads in the wells of the upper chamber that contains a membrane or frit or other device that prevents beads from passing out. Sample is added to the upper chamber and incubated with the affinity-beads, washed to remove non-specific binding, then specific binders eluted into a second microtiter plate with solid bottom.

For high resolution polypeptide fragment separation, liquid chromatography ESI-MS/MS can be used. The polypeptide fragment mixtures eluted from the affinity chromatography columns can be individually analyzed by automated LC-MS/MS using capillary reversed phase chromatography as the separation method (Yates et al., Methods Mol. Biol. 112:553-569 (1999)) and data dependent CID with dynamic exclusion (Goodlett, et al., supra, 2000) as the mass spectrometric method.

For high throughput analysis of all affinity fractions simultaneously, the high-resolution separations can be carried out in parallel. A device for this consists of parallel separation capillaries, including but not limited to CE, SPE-CE, HPLC, each terminating with a piezo-electric pump or other device capable of rapidly sampling onto a MALDI MS sampling plate. Each piezo-electric pump or similar device is capable of rapid sampling that preserves resolution from the separation and provides an increase in MALDI-TOF sensitivity by depositing the sample in a very small spot. The effluent from each separation can be deposited simultaneously onto a MALDI target or can also be deposited into a microtiter plate.

For database searching, the sequence of polypeptide fragments for which suitable CID spectra were obtained are determined by searching a sequence database from the species under investigation. A sequence database search program such as SEQUEST (Eng, J. et al., J. Am. Soc. Mass. Spectrom. 5:976-989, (1994)) or a program with similar capabilities can be advantageously used to search a database.

For polypeptide identification index construction, the sequences of all the peptides that have been identified by the procedure described above can be entered in a database and annotated with characteristics that were generated during the above-described steps. These attributes can include, for example, partial amino acid composition, the approximate molecular mass of the parent polypeptide, which can be determined, for example, by the optional fractionation step, the order of elution from a first chromatography step, the order of elution time from a second chromatography step, and the like.

Collectively, a sufficient number of characteristics can be determined that distinguish each polypeptide fragment in a polypeptide identification index. The collection of characteristics that uniquely identify a polypeptide represent a "bar code" or polypeptide identification code. Characteristics associated with an unknown polypeptide can be subsequently determined and compared to a previously generated polypeptide identification index. Alternatively, a polypeptide identification index can be determined along with the unknown polypeptide. However, the accumulation of information related to characteristics associated with a polypeptide and collection in an index is convenient for minimizing the experimental steps needed at the time of analyzing a sample. Therefore, a polypeptide identification code that is determined for a fragment of a polypeptide generated in a subsequent experiment can be used to identify a polypeptide in a sample by correlating the polypeptide identification code newly generated for an unknown polypeptide with the polypeptide identification index to identify the unknown polypeptide.

For the identification of a polypeptide by comparison with a polypeptide identification index, a set of characteristics associated with a polypeptide can be determined generally as described herein for generating a polypeptide identification index, or can be determined using an equivalent, modified or abbreviated method, or any method that allows for the determination of characteristics associated with a polypeptide. The number of characteristics sufficient to uniquely identify a polypeptide can be readily determined by those skilled in the art, as disclosed herein. The methods will generally include the identification of 2 or more characteristics, and can include 3 or more, 4 or more, 5 or more, 6 or more, 7 or more, 8 or more, 9 or more, 10 or more, 15 or more, 20 or more, 30 or more, or even 50 or more characteristics, or any number of characteristics so long as a sufficient number of characteristics are determined that distinguish each of the polypeptides in the index. The number of characteristics to include in a polypeptide identification index will depend on the particular use of the index and the complexity of the sample to be analyzed.

In generating a polypeptide identification index, characteristics associated with a polypeptide can be used to obtain the polypeptide sequence by searching a sequence database. For example, a partial amino acid sequence of a polypeptide or fragment optionally determined by mass spectrometry can be readily used to search a polypeptide or translated nucleic acid sequence database to identify a name or sequence identification number, such as an accession number, that uniquely describes a polypeptide. A polypeptide identification index can therefore contain polypeptide characteristics such as a common name, a numeric or alphanumeric identification code from a publicly available database, or any other identifying code selected for identifying a polypeptide identification code in a polypeptide identification index.

To obtain sequence information from polypeptides that do not have a parent polypeptide or nucleic acid sequence in a database or that contain an unexpected post-translational modification that makes identification difficult, de novo sequencing can be performed. Identified amino acid sequence can be used to search a polypeptide or nucleic acid sequence database as described above. *De novo* sequencing can be performed using a variety of methods. A particularly useful method of de novo sequencing involves using a MS dataset generated for polypeptide identification. Methods for sequencing polypeptides using mass spectrometry are well known to those skilled in the art (see, for example, Kinter and Sherman, Protein Sequencing and Identification Using Tandem Mass Spectrometry, John Wiley & Sons, New York (2000)).

It is understood that, although sequence information regarding a polypeptide or portion thereof, for example, determined by a method such as CID, can be included as a characteristic in a polypeptide identification index, the disclosed methods obviate the need to sequence an unknown polypeptide in order to identify it, although sequence information can be included in generating a polypeptide identification index, if desired. Accordingly, a polypeptide identification index can contain information on characteristics associated with a polypeptide that is additional to those characteristics sufficient to identify a polypeptide, for example, sequence information. By accumulating information regarding characteristics associated with a polypeptide in an index, the identity of a polypeptide can be readily determined in the absence of obtaining sequence information on the unknown polypeptide.

A chromatographic separation can be used to determine a characteristic of a polypeptide because the physicochemical properties of a polypeptide are reflected in the behavior of the polypeptide on chromatographic media. For example, a highly charged polypeptide will be eluted from an anion or cation exchange column under specific pH and/or salt conditions that differ from the pH and/or salt conditions under which an uncharged or oppositely charged polypeptide will elute. Therefore, a characteristic associated with a polypeptide can be the particular pH and/or salt condition under which the polypeptide is eluted from a chromatographic column. Similarly, conditions under which a polypeptide elutes from any type of chromatographic column can be determined. An order of elution or buffer condition at which a polypeptide is eluted from a column can be assigned a value to be annotated in a polypeptide index or to be used for comparing with corresponding values in a polypeptide index. A value can be, for example, relative position in an elution profile under defined conditions, a time of elution under a given set of conditions and flow rate, the relative time or order of elution in relation to an external standard fraction number or internal standard, salt concentration, pH, or any parameter that describes the behavior of a polypeptide on a particular chromatography column that can be reproducibly determined. Alternative methods include gel electrophoresis, for example, isoelectric focusing (IEF) or other analytical electrophoretic methods. For example, IEF was shown to be a useful characteristic for generation of a bar code for identifying a polypeptide (see Example VI). Methods for fractionating polypeptides are well known to those skilled in the art (Scopes, Protein Purification: Principles and Practice, 3rd ed.,Springer Verlag, New York (1993)). The chromatographic methods can be used in a traditional chromatography format or as a batch binding and elution method, for example, in bulk or in a multi-well format.

Protein fractionation steps are useful in the disclosed methods for both reducing the complexity of a polypeptide sample prior to mass analysis of a polypeptide or fragment thereof and for determining characteristics associated with a polypeptide. Any of the well known fractionation steps, in addition to chromatographic fractionation described above, can be used to reduce the complexity of the sample and/or serve as a determined characteristic associated with a polypeptide. Exemplary frationation steps include salt precipitation such as ammonium sulfate or precipitation with chemicals such as polyethylene glycol or polyethyleneimine, subcellular fractionation, tissue fractionation, immunoprecipitation, and the like (see Scopes, supra, 1993). A fractionation step can be used to reduce the complexity of a polypeptide population. For example, complexity reduction can be used in the isolation of a polypeptide subpopulation containing polypeptides tagged on a particular amino acid. In the case of a tissue sample, fractionation can include the isolation of one or more particular cell types, for example, by centrifugation techniques or immunoselection. Furthermore, other fractionation steps such as subcellular fractionation can also be applied to reduce the complexity of a sample and/or provide a characteristic useful for identifying a polypeptide. The fractionation steps can potentially provide biologically important information on the polypeptide, for example, whether the polypeptide is located in an organelle or is a nuclear protein, a membrane protein, and/or part of a signaling complex, and the like. Any fractionation step that advantageously reduces polypeptide population complexity can be applied in the disclosed methods

A polypeptide fractionation step is useful in the disclosed methods for determining a characteristic associated with a polypeptide. For example, a protein fractionation method based on molecular weight can be used to determine a polypeptide molecular weight. Methods such as SDS-PAGE, commercially available gel elution or preparative cell systems (BIO-RAD), and size exclusion chromatography can be used to determine the apparent molecular weight of a polypeptide or fragment. Polypeptide and/or fragment molecular weight is a characteristic that can be included in a polypeptide identification index.

The particular set of characteristics determined for a polypeptide in generating a polypeptide identification index or for identifying a polypeptide can be selected by the user and will depend on the polypeptide sample, the methods used to prepare the polypeptide sample, the method of mass spectrometry employed and the preferences of the user. The characteristics of a polypeptide can be obtained in any temporal order. For example, polypeptide characteristics can be collected in an order that provides time efficiency or convenience or can be collected as dictated by a particular method selected for sample processing.

In generating a polypeptide index, sequence information, for example, determined by CID, as well as other characteristics of a polypeptide can be used, and the sequence information is particularly useful for correlating other characteristics of a polypeptide with a particular sequence to identify the polypeptide. However, the methods are advantageous in that, once a polypeptide identification index has been generated, obtaining sequence information on a polypeptide is not required. Instead, other characteristics sufficient to identify a polypeptide can be determined, for example, masses and/or ratios between peptides as well as other characteristics, and compared to a polypeptide identification index, which itself can include sequence information, thereby.eliminating the need to sequence a polypeptide in order to identify it.

The disclosed methods for generating a polypeptide identification index involve determining a set of characteristics associated with a first and second polypeptide in which the determined characteristics are sufficient to distinguish the first and second polypeptides. Characteristics that are sufficient to distinguish the first and second polypeptides refer to a set of characteristics that can be uniquely attributed to a polypeptide so that the polypeptide identity can be determined unambiguously with the polypeptide identification index. In a case in which set of characteristics is shared by one or more polypeptides, an additional characteristic that allows a polypeptide to be distinguished from another polypeptide is determined. Thus, the polypeptides represented in a polypeptide identification index can be distinguished from each other by the set of characteristics that identify each polypeptide.

The disclosed methods for identifying a polypeptide can be applied to a population of polypeptides in which two or more polypeptides are identified and can be conveniently used to identify multiple polypeptides in a sample simultaneously, if desired. Therefore, the method can be applied to a simple or complex polypeptide sample. A simple polypeptide sample can be, for example, a purified polypeptide sample containing one to several polypeptides. A complex sample can be, for example, a cell or tissue lysate or fraction containing a few to several hundred polypeptides or even thousands or tens of thousands of polypeptides. Using the methods described herein, the determination of polypeptide characteristics can require the collection of experimental data resulting from a series of steps, such as, for example, a series of chromatographic separations.

An exemplary process useful for organizing data obtained during analysis of complex polypeptide samples involves parceling information into theoretical "bins". For example, an ICAT™ type-labeled mixture of polypeptides can be separated by size into a particular number of bins, which can be fractions eluting from chromatography column, such as size exclusion, ion exchange, and the like, or segments of an SDS polyacrylamide gel. The polypeptides in each bin can be fragmented by a sequence specific cleavage method. Alternatively, analysis of polypeptides in a sample can be performed without fractionating the polypeptides so long as there has been a sufficient reduction in complexity of the sample to allow the identification of the polypeptide without fractionation. The peptide mixture, which has been fractionated into bins, can be further fractionated by various methods, including, for example, ion exchange chromatography, affinity chromatography such as is used with the isolation of ICAT™ type labeled peptides, reverse phase liquid chromatography, or other chromatographic techniques. Each bin of peptides can then be further binned by a further fractionation step such as ion exchange chromatography and once again divided further into a particular number of bins. Each of these bins can be further separated by another fractionation step such as reverse phase chromatography and divided further into a particular number of bins, each of which can be analyzed by mass spectrometry. Hence each polypeptide analyzed by such a method will have five associated characteristics that can be represented, for example, as a 5-digit polypeptide identification code or "bar code" based on cysteine content, size, charge, hydrophobicity, and mass.

The disclosed methods for indexing characteristics associated with a large number of polypeptides use an amount of computer memory that is quadratic in sequence length. An advanced data structure such as, for example, suffix trees, can be used to reduce the requirements of computer memory (Gusfield, Algorithms on Strings, Trees and Sequences: Computer Science and Computational Biology, Cambridge University Press (1997)). Suffix trees are a compact data representation for all suffixes in a database of sequences. In their pure form, they can be constructed in linear time and stored in linear, instead of quadratic memory. Various modifications of suffix trees and traversal algorithms can be used to optimize computation time and use of computer memory associated with searching a polypeptide identification index.

A set of determined characteristics associated with a polypeptide are compared to a polypeptide identification index. Various search algorithms can be employed for matching values assigned to determined characteristics with annotated values in the index. A useful strategy for increasing the efficiency of database searching is the narrowing or "constraining" of the database. The term "constrain" when used in reference to a polypeptide identification index refers to a limitation that is applied to a polypeptide identification index in order to obtain a subindex containing a fraction of polypeptide identification codes corresponding to polypeptides having characteristics that match one or more characteristics of a polypeptide to be identified. A subindex can be generated when a group of polypeptides having a common characteristic is selected out of a polypeptide identification index or when a particular characteristic contained in a polypeptide identification code is used to omit one or more polypeptides from an index. A common characteristic can be a definite physicochemical characteristic such as a partial amino acid sequence or any other determined characteristic assigned a range of values. For example, a mass of a polypeptide fragment expressed as a range of values that account for the error in mass determination can serve as a constraint for selecting a subset of polypeptides or fragments of a particular mass.

One characteristic associated with a polypeptide that can be used to constrain a database is partial amino acid composition. The partial amino acid composition of a polypeptide includes the identification of at least a single amino acid present in a particular polypeptide or fragment thereof. A partial amino acid sequence can be obtained, for example, by treating a polypeptide or fragment thereof with a reagent that results in the generation of a polypeptide or fragment that contains one or more defined amino acids. For example, a sequence specific polypeptide cleavage method will produce fragments with one or more known amino acid residues at the fragment carboxy- or amino-terminus. However, it is not necessary to know if a specific amino acid residue is located at the fragment carboxy- or amino-terminus of a polypeptide. Accordingly, cleavage of a polypeptide with a sequence specific protease indicates the presence of the corresponding amino acid and/or sequence in the polypeptide or peptide fragment thereof. Similarly, a reagent can be used to specifically modify or label one or more specific amino acid residues of a polypeptide or fragment. A polypeptide or fragment that contains such a modification or label will be known to contain a specific amino acid. Partial amino acid composition is a characteristic associated with a polypeptide that can be useful for constraining a polypeptide identification index to generate a polypeptide identification subindex.

The comparison of a set of determined characteristics with a polypeptide identification index can therefore involve a series of searches constrained by a determined characteristic of a polypeptide. For example, an initial search of parent polypeptide or fragment mass can be performed, resulting in the generation of a polypeptide identification subindex containing polypeptide and fragment mass values that are similar to, that is, within the range of instrument error, the polypeptide or fragment thereof to be identified. A second characteristic to be searched against the generated polypeptide identification subindex, such as the presence of a cysteine residue in the polypeptide to be identified, provides a further constraint and can be used to generate a further polypeptide identification subindex.

The determined mass of a polypeptide or fragment is a characteristic that can be advantageously used to constrain such a database search to increase the efficiency of searching a large database. For example, tandem MS spectra can be analyzed using software such as SEQUEST™, which generates a list of peptides in a database that match the molecular mass of the unknown peptide on which CID was carried out and then compares the observed CID spectrum of the unknown with that for all possible isobars (Eng, J. et al., J. Am. Soc. Mass. Spectrom. 5:976-989, (1994)). Therefore, the set of peptides having a molecular mass similar to the polypeptide fragment being analyzed generated by this type of search provides a subset of possible parent polypeptides represented by the polypeptide fragment. The subset can then be searched using, for example, a partial amino acid composition, to identify the parent polypeptide. Those skilled in the art will know or can readily determine appropriate correlation score parameters for a particular search using software applications such as SEQUEST™.

A method of comparing two or more polypeptide populations employs a method for quantitatively distinguishing the two polypeptide populations, such as the method described herein using an ICAT™ type reagent and is illustrated in Figure 3. Two or several chemically identical but differentially isotopically labeled ICAT™ type reagents can be used at this step. Therefore, although Figure 3 depicts two samples, multiple samples can be compared using the methods described herein. The samples depicted in Figure 3 contain polypeptide populations harvested from the same sample type that differ from each other in growth condition. Exemplary differential growth conditions can include growth under different metabolic conditions or cells at different metabolic states, comparison of a normal and disease sample such as a tumor sample, comparison of untreated versus cells treated with a pharmacological agent, and the like.

As shown in Figure 3, the samples are independently labeled using an ICAT™ type reagent, combined, and characteristics of the polypeptides and corresponding fragments are determined, as described herein. Polypeptides and fragments generated during this process can be analyzed using single stage mass spectrometry, rather than by MS/MS, if desired, to increase sample throughput and sensitivity (Goodlett et al., *supra*, 2000). The characteristics determined for polypeptides and fragments are used to determine polypeptide identities, as described herein. Subsequently, the mass spectra can be examined for pairs of peptide ions that co-fractionated throughout the process and that have a mass difference that precisely corresponds to the mass difference encoded in the ICAT™ type reagent. The relative signal intensities of the two peaks indicate the relative abundance of the fragment polypeptides and therefore indicate the relative abundance of the corresponding parent polypeptide initially present in the sample. Therefore, a method for comparing the polypeptides contained in two polypeptide samples can involve the generation of two reference polypeptide indices that contain, for each polypeptide identified, a quantitative determination of polypeptide amount in addition to a polypeptide identification code.

An alternative method for comparing two or more polypeptide populations is the comparison of one or more polypeptide samples to a previously determined polypeptide reference index. A set of characteristics of one of more polypeptides in a polypeptide sample can be identified and compared to a reference polypeptide identification index to determine the identities of one or more polypeptides and comparative quantities of the identified polypeptides. If desired, an unknown sample can be compared to a reference sample using the above-described quantitative methods to determine relative expression levels of the polypeptides. A reference sample can be, for example, a sample from a healthy individual or a sample from a control condition useful for comparing to the physiological state of another sample such as a disease sample.

A polypeptide identification index that contains quantitative determinations of polypeptide amount is considered to be a "polypeptide profile" of the particular sample used to generate the index. A polypeptide profile, as used herein, is a set of polypeptide identification codes that includes polypeptide amount, generated for a specific sample.

A polypeptide profile is useful in methods of proteomics because such a profile can be used to distinguish between different conditions or states of cells, tissues, organs, and organisms. The polypeptides expressed by a cell or tissue at a particular time can be used to define the state of the cell or tissue at the time of measurement. Therefore, quantitative and qualitative differences between the polypeptide profiles of the same cell type in different states can be used to diagnose the respective states. Examples for such comparisons include normal versus tumor cells, cells at different metabolic states and untreated cells versus cells treated with specific pharmacological agents. The differences between two polypeptide profiles can be described as a "differential polypeptide profile". A differential polypeptide profile is useful for analyzing quantitative changes in the polypeptides contained in samples derived from different cell types such as, for example, cancerous and normal cells, stimulated and unstimulated cells, or from different tissue samples of clinical interest.

The disclosed methods for generating differential polypeptide profiles are applicable to the analysis of changes in the polypeptide profiles in samples such as body fluids. A differential polypeptide profile is determined by comparing the polypeptide profile of two specimens, for example, a normal to disease-related polypeptide profile. For example, a polypeptide profile representative of a normal specimen state can be generated and compared to a specimen suspected to be in an abnormal or disease state. Alternatively, a reference polypeptide profile representative of a disease state can be compared with a specimen from an individual having or suspected of having a particular disease state. A reference polypeptide profile representative of a normal or disease state can be determined using a specimen from a particular individual or a population of individuals.

If desired, analysis can be performed on a population rather than an individual, particularly a reference population or control population. Such a reference population can be used for comparison of an unknown sample. One skilled in the art can determine an appropriate reference population based on the particular application of the disclosed methods. The methods can be used to generate a differential polypeptide profile that identifies the differences in polypeptide expression between two samples, for example, a normal and disease state. The size of the reference population depends on the criteria used to select reference individuals. Depending on the selection criteria and particular application of the disclosed methods , a reference population can be a relatively small number to a large number of individuals, including thousands of individuals.

The large-scale analysis of samples from patients having specifically diagnosed diseases or exhibiting signs or symptoms of a disease is useful for identifying clinical markers or constellations of markers for the respective conditions. Samples from an individual having a disease can be used to generate a qualitative and/or quantitative polypeptide identification index for that disease. Similarly, the comparative analysis of polypeptides contained in samples from patients undergoing therapeutic treatment can be used to identify diagnostic markers or constellation of markers indicating the success or failure of the treatment. The methods are also applicable to the analysis of such samples on a systematic, population-wide scale for the discovery or screening of markers or constellations of markers useful for indicating the predisposition of individuals for certain clinical conditions.

A method for generating a polypeptide identification index is disclosed. The method includes the steps of (a) determining a set of two or more characteristics associated with a first polypeptide, or a peptide fragment thereof, one of the characteristics being the mass of a peptide fragment of the polypeptide, the peptide fragment mass being determined by mass spectrometry; (b) repeating step (a) for a second polypeptide; (c) optionally determining one or more additional characteristics associated with the first and second polypeptides, wherein the determined characteristics are sufficient to distinguish the first and second polypeptides, thereby generating a polypeptide identification index for the first and second polypeptides. The method can further comprise repeating steps (a) through (c) one or more times for a different polypeptide, wherein the determined characteristics are sufficient to distinguish each of the polypeptides, thereby generating a polypeptide identification index for each of the polypeptides. As with determining characteristics of a polypeptide, the polypeptide identification index can be determined with any of the methods disclosed herein or any well known methods for determining characteristics associated with a polypeptide. The polypeptide identification index can optionally be obtained by determining mass in the absence of ion selection.

The disclosed methods for generating a polypeptide identification index can involve the determination of polypeptide or fragment mass in the absence of ion selection for producing fragment ions and can further involve the determination of a fragment mass at an accuracy of greater than 1 part per million, or even lower accuracy (higher ppm), if desired. Although a polypeptide identification index can contain a polypeptide amino acid sequence, it is not required that a polypeptide or fragment be sequenced for practicing the disclosed methods for generating a polypeptide identification index or identifying a polypeptide using a polypeptide identification index.

A method for identifying a polypeptide is disclosed. The method includes steps of (a) simultaneously determining the mass of a subset of parent polypeptides from a population of polypeptides and the mass of peptide fragments of the subset of parent polypeptides; (b) comparing the determined masses to a polypeptide identification index; and (c) identifying one or more polypeptides of the polypeptide identification index having the determined masses. The polypeptide identification index can be an annotated polypeptide index. The method can further comprise the steps of (d) determining one or more additional characteristics associated with one or more of the parent polypeptides; (e) comparing the characteristics determined in step (a) and step (d) to the polypeptide identification index; and (f) optionally repeating steps (d) and (e) one or more times, wherein a set of characteristics is determined that identifies a parent polypeptide as a single polypeptide in the polypeptide identification index. The methods can further include quantitating the amount of identified polypeptide in a sample containing the polypeptide. As disclosed herein, the above method, as well as other disclosed methods, can be performed at particular mass accuracies. Identification of a parent polypeptide as a single polypeptide in a polypeptide identification index refers to determining a sufficient number of characteristics so that a particular polypeptide in the polypeptide identification index matches the determined characteristics, that is, a polypeptide is identified.

The disclosed method for identifying a polypeptide includes a step of simultaneously determining the mass of a subset of parent polypeptides from a population of polypeptides and the mass of polypeptide fragments of the subset of parent polypeptides (see Example III). The simultaneous determination of masses of a subset of parent polypeptides refers to the acquisition of a subset of parent polypeptide mass values from a single sample containing a polypeptide population. The term "simultaneous" is intended to mean that the masses of parent polypeptides and polypeptide fragments are determined concurrently such that the MS method used can acquire masses of parent polypeptides and corresponding fragments in a time frame sufficient that parent and fragment masses can be correlated to the same subset of polypeptides. For example, the polypeptides being sampled in a MS method will change over time as different subsets of polypeptides elute from a chromatographic column as dictated by the flow rate of the column. A simultaneous determination occurs during a time period before a particular subset of polypeptides is altered due to the introduction of an additional polypeptide or loss of a polypeptide of the polypeptide subset that occurs as a result of on-line sampling methods.

Simultaneous determination of the mass of a subset of polypeptides can be performed, for example, in the absence of selection of a single ion for mass determination. For example, several polypeptides can be selected rather than a single ion (Masselon et al., Anal, Chem. 72:1918-1924 (2000)). In disclosed methods, preferably greater than 5 ions, for example, 6 ions, 7 ions, 8 ions, 9 ions, 10 ions, or even greater numbers of ions are selected. In such a case, the polypeptide identification index is preferably an annotated polypeptide index.

Alternatively, simultaneous determination of masses of a subset of polypeptides can be performed in the absence of single ion selection or in the absence of ion selection in a source region (see Figure 2). In such a case, the fragment ions obtained are deconvoluted to determine which ions are associated with a particular parent polypeptide and therefore useful as a characteristic associated with the parent polypeptide. Such a method can be useful for detecting and identifying less abundant ions that are not selected for fragmentation in standard MS methods.

A mass spectrometry method useful for obtaining polypeptide and polypeptide fragment masses simultaneously is in-source CID on ESI-TOF. The method involves continuously alternating between parent ion and in-source CID scans. In-source CID scans provide specific fragment ions traceable to a given parent ion even in the presence of multiply fragmented parent ions. Parent-fragment ion lineages can be determined by deconvolution of mass spectrometry data using appropriate software. MS instruments providing lower accuracy measurements, for example, ESI-TOF, can be used advantageously for providing unique constraints for polypeptide identification.

A method for identifying a polypeptide is disclosed. The method includes the steps of (a) determining two or more characteristics associated with the polypeptide, or a fragment thereof, one of the characteristics being mass of a fragment of the polypeptide, the fragment mass being determined by mass spectrometry in the absence of ion selection for producing fragment ions; (b) comparing the characteristics associated with the polypeptide to a polypeptide identification index; and (c) identifying one or more polypeptides in the polypeptide identification index having the characteristics. The method can further comprise the steps of (d) determining one or more additional characteristics associated with the polypeptide; and (e) comparing the characteristics determined in step '(a) and step (d) to the polypeptide identification index. As with other disclosed methods the method can include quantitating the amount of the identified polypeptide in a sample. The methods can also be performed at a particular mass accuracy.

A method for identifying a polypeptide is disclosed. The method includes the steps of (a) determining two or more characteristics associated with the polypeptide, or a fragment thereof, one of the characteristics being mass of a fragment of the polypeptide, the fragment mass being determined by mass spectrometry at an accuracy in ppm of greater than 2.5 ppm; (b) comparing the characteristics associated with the polypeptide to a polypeptide identification index; and (c) identifying one or more polypeptides in the polypeptide identification index having the characteristics. The method can further include the steps of (d) determining one or more additional characteristics associated with the polypeptide; and (e) comparing the characteristics determined in step (a) and step (d) to the polypeptide identification index. The methods can also be performed at lower accuracy (higher ppm).

As disclosed herein, parent proteins were identified from a combination of (1) cysteine content, (2) accurate peptide mass and (3) accurate peptide fragment mass without selection of a specific ion (see Example IV). Although cysteine content was used in this particular example, it is not required that cysteine content be a determined characteristic. In contrast to traditional tandem MS, all peptides entering the MS together were fragmented without selection of an individual ion. All fragment ions for the two peptides simultaneously fragmented were measured together, in contrast to tandem MS, where fragment ions are measured for a single peptide at a time.

The disclosed methods involve determining characteristics associated with a polypeptide. A sample containing a polypeptide can be as simple as an isolated polypeptide mixture containing a polypeptide or as complex as a sample containing essentially all of the polypeptides expressed in an organism. Furthermore, a sample can be fractionated, if desired, using the methods disclosed herein.

A polypeptide can be in a sample isolated from a variety of sources. For example, a polypeptide sample can be prepared from any biological fluid, cell, tissue, organ or portion thereof, or any species of organism. A sample can be present in an individual and obtained or derived from the individual. For example, a sample can be a histologic section of a specimen obtained by biopsy, or cells isolated from an individual that are placed in or adapted to tissue culture. A sample further can be a subcellular fraction or extract. A sample can be prepared by methods known in the art suitable for maintaining polypeptide solubility, such as those described herein.

A specimen refers specifically to a sample obtained from an individual. A specimen can be obtained from an individual as a fluid or tissue specimen. For example, a tissue specimen can be obtained as a biopsy such as a skin biopsy, tissue biopsy or tumor biopsy. A fluid specimen can be blood, serum, urine, saliva, cerebrospinal fluid or other bodily fluids. A fluid specimen is particularly useful in the disclosed methods since fluid specimens are readily obtained from an individual. Methods for collection of specimens are well known to those skilled in the art (see, for example, Young and Bermes, in Tietz Textbook of Clinical Chemistry, 3rd ed., Burtis and Ashwood, eds., W.B. Saunders, Philadelphia, Chapter 2, pp. 42-72 (1999)).

A polypeptide to be used in the disclosed methods can be obtained from a source such as a cell, tissue, organ or organism. A variety of methods are known in the art for lysing a cell. Cells can be lysed, for example, by denaturants, one or more cycles of freezing and thawing, sonication, and the like. Following lysis, the polypeptide mixture can be subjected to a fractionation step to remove, for example, nucleic acid or lipid, or to remove intact subcellular fractions or organelles. Methods of lysing and fractionating cells are well known to those skilled in the art (see Scopes, *supra,* 1993).

For identification of a polypeptide, a sample or specimen can be contained in a buffer suitable for maintaining polypeptide solubility such as, for example, a buffer containing a detergent, including denaturants such as sodium dodecyl sulfate (SDS). Denaturants useful for solubilizing polypeptides include, for example, guanidine-HCl, guanidine-isothiocyanate, urea and the like. In the case of guanidine-isothiocyanate, as with treatment with any reagent that can covalently modify a polypeptide, such reagents can be used so long as the polypeptide identification index to which the sample is to be compared has been prepared in substantially the same manner as the sample sufficient for comparison of the same polypeptide. Other denaturants well known in the art can be similarly used for solubilizing polypeptides. In addition, reducing agents such as dithiothreitol (DTT), dithioerythritol (DTE), or mercaptoethanol can be included.

The disclosed methods can optionally involve protein fractionation steps. Protein fractionation refers to any method useful for removing one or more polypeptides from a polypeptide population. Fractionation can include, for example, a centrifugation step that separates soluble from insoluble components, a method of electrophoresis, a method of chromatography, or any of the methods disclosed. For chromatographic separation, a wide variety of chromatographic media well known in the art can be used to separate polypeptide populations. For example, polypeptides can be separated based on size, charge, hydrophobicity, binding to particular dyes and other moieties, including affinity ligands, associated with chromatographic media. Size exclusion, gel filtration and gel permeation resins are useful for polypeptide separation based on size. Examples of chromatographic media for charge-based separation are strong and weak anion exchange and strong and weak cation exchange resins. Hydrophobic or reverse phase chromatography can also be used.

Affinity chromatography can also be used including, for example, dye-binding resins such as Cibacron blue, substrate analogs, including analogs of cofactors such as ATP, NAD, and the like, ligands, specific antibodies, either polyclonal or monoclonal, and the like. An exemplary affinity resin includes affinity resins that bind to specific moieties that can be incorporated into a polypeptide such as an avidin resin that binds to a biotin tag on a polypeptide, as disclosed herein. The resolution and capacity of particular chromatographic media are known in the art and can be determined by those skilled in the art. The usefulness of a particular chromatographic separation for a particular application can similarly be assessed by those skilled in the art.

Those of skill in the art will be able to determine the appropriate chromatography conditions for a particular sample size or composition and will know how to obtain reproducible results for chromatographic separations under defined buffer, column dimension, flow rate, temperature, and other conditions. Protein fractionation methods can optionally include the use of an internal standard, for example, to assess the reproducibility of a particular chromatographic application or other fractionation step. Appropriate internal standards will vary depending on the chromatographic medium or fractionation step. Those skilled in the art will be able to determine an internal standard applicable to a method of chromatography or fractionation step.

Polypeptide tagging is useful in the disclosed methods for reducing polypeptide sample complexity, providing a database search constraint, and enabling quantitative polypeptide comparisons. The complexity of a polypeptide sample can be reduced by tagging a polypeptide with an affinity tag that can be used for isolating a subpopulation of polypeptides that contain the tag. For example, a population of polypeptides and fragments can be labeled on a relatively rare amino acid, such as cysteine, or based on a post-translational modification, and a subpopulation of polypeptides and fragments containing the tag can be isolated. The subpopulation of polypeptides and fragments isolated by labeling a particular amino acid will thus contain a known amino acid. As described herein, a known amino acid constitutes a partial amino acid composition which is useful for constraining a database search. Quantitative polypeptide comparisons can be performed by differentially tagging two polypeptides or polypeptide populations. An ICAT™ type affinity reagent or IDEnT type reagent, described in more detail below, is particularly useful for this purpose, although any other method of polypeptide tagging can be similarly applied for polypeptide comparisons.

Polypeptide tagging can be performed using a variety of methods known in the art. A reagent for polypeptide tagging or modification can contain various components that are separated by linker regions. Components of a polypeptide tagging reagent can include a reactive group that modifies a specific chemical group of a polypeptide, a moiety that can be detected, such as by mass spectrometry, and an affinity tag to be used for polypeptide isolation. Two examples of polypeptide tagging reagents, ICAT™ type and IDEnT, are described in detail below, although any type of polypeptide tag can be used, if desired.

The disclosed methods for quantitatively comparing two polypeptide populations can involve the use of the isotope-coded affinity tag (ICAT™) method (Gygi et al., Nature Biotechnol. 17:994-999 (1999); WO 00/11208, . An ICAT™ type reagent can additionally be useful for polypeptide tagging applications that do not involve quantitative comparisons. The ICAT™ type reagent method uses an affinity tag that can be differentially labeled with an isotope that is readily distinguished using mass spectrometry, for example, hydrogen and deuterium. The ICAT™ type affinity reagent consists of three elements, an affinity tag, a linker and a reactive group.

One element of the ICAT™ type affinity reagent is an affinity tag that allows isolation of peptides coupled to the affinity reagent by binding to a cognate binding partner of the affinity tag. A particularly useful affinity tag is biotin, which binds with high affinity to its cognate binding partner avidin, or related molecules such as streptavidin, and is therefore stable to further biochemical manipulations. Any affinity tag can be used so long as it provides sufficient binding affinity to its cognate binding partner to allow isolation of peptides coupled to the ICAT™ type affinity reagent. An affinity tag can also be used to isolate a tagged peptide with magnetic beads or other magnetic format suitable to isolate a magnetic affinity tag. In the ICAT™ type reagent method, or any other method of affinity tagging a peptide, the use of covalent trapping can be used to bind the tagged peptides to a solid support, if desired.

A second element of the ICAT™ type affinity reagent is a linker that can incorporate a stable isotope. The linker has a sufficient length to allow the reactive group to bind to a specimen polypeptide and the affinity tag to bind to its cognate binding partner. The linker also has an appropriate composition to allow incorporation of a stable isotope at one or more atoms. A particularly useful stable isotope pair is hydrogen and deuterium, which can be readily distinguished using mass spectrometry as light and heavy forms, respectively. Any of a number of isotopic atoms can be incorporated into the linker so long as the heavy and light forms can be distinguished using mass spectrometry. Exemplary linkers include the 4,7,10-trioxa-1,13-tridecanediamine based linker and its related deuterated form, 2,2',3,3',11,11',12,12'-octadeutero-4,7,10-trioxa-1,13-tridecanediamine, described by Gygi et al. (*supra*, 1999). One skilled in the art can readily determine any of a number of appropriate linkers useful in an ICAT™ type affinity reagent that satisfy the above-described criteria.

The third element of the ICAT™ type affinity reagent is a reactive group, which can be covalently coupled to a polypeptide in a specimen. Methods for modifying side chain amino acids in polypeptides are well known to those skilled in the art (see, for example, Glazer et al., Laboratory Techniques in Biochemistry and Molecular Biology: Chemical Modification of Proteins, Chapter 3, pp. 68-120, Elsevier Biomedical Press, New York (1975): Pierce Catalog (1994), Pierce, Rockford IL). Any of a variety of reactive groups can be incorporated into an ICAT™ type affinity reagent so long as the reactive group can be covalently coupled to a polypeptide. For example, a polypeptide can be coupled to the ICAT™ type affinity reagent via a sulfhydryl reactive group, which can react with free sulfhydryls of cysteine or reduced cystines in a polypeptide. An exemplary sulfhydryl reactive group includes an iodoacetamido group, as described in Gygi et al. (*supra,* 1999). Other exemplary sulfhydryl reactive groups include maleimides, alkyl and aryl halides, α-haloacyls and pyridyl disulfides. If desired, the polypeptides can be reduced prior to reacting with an ICAT™ type affinity reagent, which is particularly useful when the ICAT™ type affinity reagent contains a sulfhydryl reactive group.

A reactive group can also react with amines such as Lys, for example, imidoesters and N-hydroxysuccinimidyl esters. A reactive group can also react with carboxyl groups found in Asp or Glu, or the reactive group can react with other amino acids such as His, Tyr, Arg, and Met. A reactive group can also react with a phosphate group for selective labeling of phosphopeptides, or with other covalently modified peptides, including glycopeptides, lipopeptides, or any of the covalent polypeptide modifications disclosed herein. One skilled in the art can readily determine conditions for modifying specimen molecules by using various reagents, incubation conditions and time of incubation to obtain conditions optimal for modification of specimen molecule for use in the disclosed methods.

The ICAT™ type reagent method is based on derivatizing a specimen molecule such as a polypeptide with an ICAT™ type affinity reagent. For comparison of two samples and/or quantitation, a control reference specimen and a specimen from an individual to be tested are differentially labeled with the light and heavy forms of the ICAT™ type affinity reagent. The derivatized specimens are combined, and the derivatized molecules cleaved to generate fragments. For example, a polypeptide molecule can be enzymatically cleaved with one or more proteases into peptide fragments. Exemplary proteases useful for cleaving polypeptides include trypsin, chymotrypsin, pepsin, papain, *Staphylococcus* aureus (VB) protease, and the like. Polypeptides can also be cleaved chemically, for example, using CNBr, acid or other chemical reagents.

Once cleaved into fragments, the tagged fragments derivatized with the ICAT™ type affinity reagent are isolated via the affinity tag, for example, biotinylated fragments can be isolated by binding to avidin in a solid phase or chromatographic format. If desired, the isolated, tagged fragments can be further fractionated using one or more alternative separation techniques, including ion exchange, reverse phase, size exclusion affinity chromatography and the like, or electrophoretic methods, including isoelectric focusing. For example, the isolated, tagged fragments can be fractionated by high performance liquid chromatography (HPLC), including microcapillary HPLC.

The fragments are analyzed using mass spectrometry (MS). Because the specimen molecules are differentially labeled with light and heavy affinity tags, the peptide fragments can be distinguished on MS, allowing a side-by-side comparison of the relative amounts of each peptide fragment from the control reference and test specimens. If desired, MS can also be used to sequence the corresponding labeled peptides, allowing identification of molecules corresponding to the tagged peptide fragments.

An advantage of the ICAT™ type reagent method is that the pair of peptides tagged with light and heavy ICAT™ type reagents are chemically identical and therefore serve as mutual internal standards for accurate quantification (Gygi et al., *supra*, 1999). Using MS, the ratios between the intensities of the lower and upper mass components of pairs of heavy- and light-tagged fragments provides an accurate measure of the relative abundance of the peptide fragments. Thus, the ICAT™ type reagent method can be conveniently used to identify differentially expressed polypeptides, if desired.

An IDEnT reagent can be used to modify a polypeptide by introducing an isotopic tag at a specific protein functional group. An exemplary IDEnT reagent is described in Goodlett et al., *supra,* 2000. An IDEnT reagent contains at least one element with an isotopic distribution that creates a unique signature in a mass spectrometer. For example, an IDEnT reagent can contain chlorine, deuterium, or another element, including a radioactive element. An IDEnT reagent can be designed to bind to a low abundance amino acid in a polypeptide, such as cysteine. The labeling of a polypeptide with an IDEnT tag can be applied to the disclosed methods by providing a constraint for searching a polypeptide identification index with polypeptide fragment masses.

Protein cleavage or fragmentation is useful in the disclosed methods for providing a constraint for database searching. Polypeptide fragmentation can be sequence-specific or non-specific. Sequence-specific polypeptide cleavage provides the advantage of obtaining polypeptide fragments that contain known amino acids, which can be used to constrain a database search. Examples of reagents useful for performing non-specific polypeptide cleavage are papain, pepsin and protease Sg. These proteases can be used to achieve a desired degree of protein fragmentation, such as, for example, the generation of about two to four polypeptide fragments from a polypeptide by altering the reaction conditions. Conditions for using these proteases are well known in the art. Examples of reagents useful for performing sequence-specific polypeptide cleavage are trypsin, V-8 protease, o-iodosobenzoic acid, cyanogen bromide and acid.

A polypeptide identification index for identifying a polypeptide from a population of polypeptides is disclosed. The index comprises an annotated set of characteristics associated with polypeptides in the index, one of the characteristics being the mass of a fragment of the polypeptide, the fragment mass being determined by mass spectrometry in the absence of ion selection for producing fragment ions. The characteristics are sufficient to distinguish one of the polypeptides from other polypeptides in the index. A polypeptide identification index can comprise characteristics for 2 or more, 3 or more, 5 or more, 10 or more, 20 or more, 50 or more, 100 or more, 150 or more, 200 or more, 500 or more, 1000 or more, 2000 or more, 5000 or more, or even 10,000 or more polypeptides. A polypeptide identification index can also include substantially all of the polypeptides in a sample. For example, a polypeptide identification index can include substantially all of the polypeptides expressed in a genome, such as a viral, bacterial, plant, or animal genome, including a mammalian genome such as human, non-human primates, mouse, rat, bovine, goat, rabbit, or other mammalian species. The number of polypeptides in a polypeptide identification index will depend on the needs of the user and will vary depending on the source of the sample to be used to identify polypeptides and the complexity of polypeptide expression in the sample. An example of generation of a polypeptide identification index for *Drosophila melanogaster* is disclosed herein (see Example V).

The polypeptide identification index can be directed to a whole organism or to particular tissues or cells in an organism or to specific subcellular fractions, for example, organelles, as desired. Accordingly, similar to the reduction in complexity applied to a sample to be tested, a polypeptide identification index directed to a particular target such as an organism, tissue, cell or subcellular fraction, can be useful for simplifying a search for identification of a particular polypeptide in a particular application. For example, in a particular diagnostic application where expression of a particular polypeptide or group of polypeptides, or the amount of expression of the polypeptides, is correlated with a particular condition such as a disease condition, the use of a polypeptide identification index directed to a relevant target can be used. For example, if a group of nuclear proteins are known to be overexpressed in a cancer cell, the use of a polypeptide identification index directed to nuclear proteins can be used to test for overexpression of the nuclear proteins in a sample from an individual using the quantitative methods disclosed herein. Moreover, the generation of a targeted polypeptide identification index and comparison to a relevant disease sample can be used to identify aberrantly expressed polypeptides, which in turn can be used in diagnostic applications, as disclosed herein.

A polypeptide identification index is disclosed comprising an annotated set of characteristics associated with polypeptides of the index comprising two or more characteristics associated with polypeptides of the index, or a fragment thereof, one of the characteristics being the mass of a fragment of the polypeptide, the fragment mass being determined by mass spectrometry in the absence of ion selection for producing fragment ions, and wherein the mass is determined at an accuracy in ppm of greater than 1 ppm.

If desired, a polypeptide identification index can be conveniently stored on a computer readable medium. Accordingly, a computer readable medium is disclosed comprising a polypeptide identification index, for example, an annotated polypeptide index. Such a computer readable medium comprising a polypeptide identification index is useful for comparing the characteristics of a polypeptide with the polypeptide identification index, which can be conveniently performed on a computer apparatus. The use of a computer apparatus is convenient since a polypeptide identification index can be conveniently stored and accessed for comparison to characteristics and/or quantitative amounts of a polypeptide in a sample. A polypeptide identification index can be conveniently accessed using appropriate hardware, software, and/or networking, for example, using hardware interfaced with networks, including the internet.

By using various hardware, software and network combinations, the disclosed methods including the step of comparing the characteristics determined for a polypeptide to a polypeptide identification index can be conveniently performed in a variety of configurations. Accordingly, computer apparatus is disclosed for carrying out computer executable steps corresponding to steps of the disclosed methods. For example, a single computer apparatus can contain instructions for carrying out the computer executable step(s) of comparing characteristics determined for polypeptide to a polypeptide identification index, a polypeptide identification index, and instructions for determining whether the characteristics determined for the polypeptide correspond to one or more polypeptides in the polypeptide identification index.

Alternatively, the computer apparatus can contain instructions for carrying out the steps of a disclosed method while the polypeptide identification index is stored on a separate medium. In addition, instructions for determining whether a polypeptide corresponds to one or more polypeptides in the polypeptide identification index can be contained on a separate computer apparatus or separate medium, or combined with the computer apparatus containing the computer executable steps of the method and/or the database on a separate medium. Such a separate computer readable medium can be another computer apparatus, a storage medium such as a floppy disk, Zip disk or a server such as a file-server, which can be accessed by a carrier wave such as an electromagnetic carrier wave. Thus, a computer apparatus containing a polypeptide identification index or a file-server on which the polypeptide identification index is stored can be remotely accessed via a network such as the internet. One skilled in the art will know or can readily determine appropriate hardware, software or network interfaces that allow interconnection of a computer apparatus.

If desired, algorithms can be used to optimize and increase the efficiency of identifying a polypeptide using a polypeptide identification index. For example, as described above, a subindex of a polypeptide identification index can be determined, and the subindex used to further identify a polypeptide by reducing the size of the database to be compared. Such a reduction by using a subindex provides a constraint that can reduce the computational time for database searching. One skilled in the art will recognize various methods to increase the efficiency of a database search.

An algorithm can be used to improve the efficiency of analysis of mass spectral data. For example, the presence of complementary ions in a tandem mass spectrum can be used to develop algorithms that more accurately calculate parent ion mass. This will improve scoring and decrease search time by narrowing peptide mass tolerance to a smaller subset of isobaric database peptides. To increase proteome coverage, ESI-MS/MS on ion traps can be conducted such that explicit peptide charge state determination is precluded, forcing all peptide tandem mass spectra to be searched twice, as [M + 2H]2+ and [M + 3H]3+. The charge state generating the best score is retained and the other discarded. An algorithm to determine charge state prior to database searching can be used to increase throughput. A routine to measure spectral quality can be used to allow cataloging of spectra prior to and post database searching. This will increase throughput by discarding poor spectra pre-search and allow follow up analysis on "good quality" spectra that failed, perhaps because of an unexpected post-translational modifications, to generate a good database match, as disclosed herein.

### EXAMPLE I

### Generation of an Annotated Polypeptide Index

This example describes the generation of an annotated polypeptide index and use of the annotated polypeptide index to identify a polypeptide in a sample.

The elements of an annotated polypeptide (AP) index, also referred to as an annotated peptide tag (APT) index or database, are the sequences of essentially all the peptides or selected peptides with specific structural features that are generated by sequence specific chemical or enzymatic fragmentation of the proteins produced by the species, cell or tissue under investigation. Each peptide is annotated with attributes, or characteristics, that are easily determined experimentally and that permit the unambiguous correlation between the annotated peptide and the protein from which the peptide originated.

The generation of an exemplary AP index can involve the following specific steps: harvest proteins; label proteins with an isotope coded affinity tag (ICAT™) type reagent; fractionate proteins by molecular weight; digest proteins with a protease, for example, trypsin, to generate peptides; separate peptides by chromatography, for example, ion exchange chromatography; purify each ion exchange fraction by affinity chromatography, for example, based on the ICAT™ type affinity tag; analyze each affinity chromatography fraction by LC/MS/MS or CE/MS/MS; identify essentially all expressed proteins via a database search of individual MS/MS peptide spectra; and generate a database of annotated peptide tags that constitute a unique bar code for an individual peptide based on measured physicochemical properties and thus the parent protein of that peptide.

The AP index can be generated as follows:(i) protein sample preparation; (ii) protein tagging; (iii) optional protein fractionation; (iv) protein fragmentation; (v) peptide separation; (vi) affinity isolation of tagged peptides; (vii) high resolution peptide separation; (viii) database searching; (ix) AP index (APT database) construction.
(i) Protein Sample Preparation. Protein samples for which quantitative proteome analysis is to be performed, for example, cells, tissues, subcellular fractions, body fluids, cellular secretions, and the like, are isolated from the respective sources using standard protocols for maintaining the solubility of the proteins.
(ii) Protein tagging. The proteins in the sample are completely denatured, reduced, and the all the sulfhydryl groups representing the side chains of reduced cysteine residues are covalently derivatized with the light or heavy form, respectively, of sulfhydryl-specific ICAT™ type reagents using the conditions described previously (Gygi et al., Nature Biotechnol. 17:994-999 (1999))(see Figure 3). While cysteine tagging is a particularly useful implementation of the method, any other chemical reaction with specificity for a chemical group in the protein can also be applied.
(iii) Optional Protein Fractionation. The mixture of tagged proteins is fractionated using any one of the known standard protein separation procedures. The applied procedure is reproducible, maintains the proteins in solution, and has a high sample capacity. A particularly useful method is preparative sodium dodecyl sulfate-polyacrylamide gel electrophoresis (SDS-PAGE).
(iv) Protein Fragmentation. The proteins in the sample mixture, or the proteins contained in each fraction if optional sample fractionation is employed, are subjected to sequence specific cleavage. A particularly useful method is protease cleavage, for example, with trypsin.
(v) Peptide Separation. The resulting peptide mixtures are subjected to a first dimension peptide separation. The peptide separation method has a high sample capacity, at least moderate resolving power, and generates highly reproducible separation patterns, irrespective of the complexity of the sample applied. A particularly useful first dimension separation method is ion exchange chromatography such as cation or anion exchange.
(vi) Affinity Isolation of Tagged Peptides. Peptides tagged with the ICAT™ type reagent, for example, cysteine containing peptides or peptides containing other amino acids or post-translational modification that can be affinity labeled, are isolated from each chromatographic fraction using avidin or streptavidin affinity chromatography. A particularly useful affinity medium is monomeric avidin immobilized on polymer beads. If ICAT™ type reagents with affinity tags different from biotin are used, affinity media complementary to that tag are used.
(vii) High Resolution Peptide Separation. A particularly useful method for high resolution peptide separation is liquid chromatography ESI-MS/MS. The peptide mixtures eluted from the affinity chromatography columns are individually analyzed by automated LC-MS/MS using capillary reversed phase chromatography as the separation method (Yates et al., Methods Mol. Biol. 112:553-569 (1999)) and data dependent CID with dynamic exclusion (Goodlett et al., Anal. Chem. 15:1112-1118 (2000)) as the mass spectrometric method.
(viii) Database Searching. The sequence of all the peptides for which suitable CID spectra are obtained is determined by searching a sequence database from the species under investigation. A particularly useful sequence database is a database containing essentially all the complete protein sequences that can be potentially expressed by the species under examination. A sequence database search program that can be used is the SEQUEST™ program (Eng, J. et al., J. Am. Soc. Mass. Spectrom. 5:976-989, (1994)) or a program with similar capabilities.
(ix) AP index (APT database) Construction. The sequences of all the peptides that have been identified by the procedure described above are entered in a database and annotated with the characteristics, or attributes, that were generated during steps (i)-(viii) above. These characteristics, or attributes, include, but are not limited to: partial amino acid composition (such as the presence of a cysteine residue in each selected peptide; see Goodlett et al., *supra*, 2000); the approximate molecular mass of the parent protein (as determined by the optional SDS-PAGE fractionation); the order of elution or elution time from an ion exchange column; the elution time from the reversed-phase column, and any other determined characteristics. Collectively, these attributes are unique for every peptide in the database akin to a bar code for each peptide. Therefore, if the same bar code is being determined from the peptides generated in subsequent experiments, they will uniquely identify the peptides generated by the experiment, simply by correlating the bar codes generated by the experiment with the bar codes present in the AP index (APT database).

For correlation of polypeptides with the AP index (APT database), the peptide samples generated for quantitative proteome analysis by the method described above are generated, treated and processed precisely like the peptides generated for the AP index (APT database), with the following exceptions. (i) The proteins in the two (or more) samples to be compared are labeled with differentially isotopic labeled ICAT™ type reagents. Two or several chemically identical but differentially isotopically labeled ICAT™ type reagents can be used at this step. (ii) The generated peptides are analyzed by single stage mass spectrometry only, rather than by MS/MS. Mass analyzers will generally have a high mass accuracy, high sensitivity and high mass resolution. Instruments with these characteristics include, but are not limited to, MALDI-TOF mass spectrometers, ESI-TOF mass spectrometers and Fourier transform ion cyclotron mass analyzers (FT-ICR-MS). The attributes determined from each peptide by this process (all the attributes determined as described above or a selection thereof) are translated into a bar code for each peptide, and the experimentally determined bar code is correlated with the bar codes from the AP index (APT database), resulting in the unambiguous identification of the peptide and therefore the protein from which the peptide originated. Subsequently, the mass spectra are examined for pairs of peptide ions that co-fractionated throughout the process and that have a mass difference that precisely corresponds to the mass difference encoded in the ICAT™ type reagent used. The relative signal intensities of the two peaks indicate the relative abundance of the peptides and therefore the relative abundance of the corresponding proteins initially present in the sample. Consequently, the correlation of the experimentally determined data with the AP index (APT database) allows quantification and identification of the proteins in the samples analyzed.

### EXAMPLE II

### Generation of a Yeast Annotated Polypeptide Index

This example describes the generation of an annotated polypeptide index for yeast.

At least 5 mg of total protein was estimated to be required at current mass spectrometer sensitivity to detect low abundance proteins using the LC/LC/MS/MS method (Gygi et al., Proc. Natl. Acad. Sci. USA 97:9390-9395 (2000)), and this amount was essentially experimentally confirmed. Gygi et al., *supra*, 2000 also demonstrated that the "binning" process is adequate for the detection of low abundance proteins and has sufficient sample capacity to accommodate the relatively large amounts of total sample.

For the construction of the database, the following procedure is used. For protein labeling, a protein sample is generated in 0.5% SDS, 50 mM Tris, pH 8.3, 5 mM ethylenediaminetetraacetic acid (EDTA) at a protein concentration of 5 mg/ml. A total of 25 mg of total yeast protein is used. Once proteins are in solution, the SDS concentration is lowered by diluting the sample 1:10 with water and adding EDTA to maintain a 5 mM EDTA concentration. The final concentration is 0.05% SDS, 5 mM Tris, 5 mM EDTA. The sample is then boiled for 3-5 min at 100°C and then chilled. Reduction of disulfide bonds is accomplished by adding sufficient Tributylphosphine (TBP) to achieve 5 mM in the sample solution. This is followed by an incubation of the sample at 37°C for 30 min. To the reduced sample, the alkylating reagent (for example, ICAT™ type reagent) is added at an estimated 5x molar excess over the SH groups present in the sample. The alkylation reaction is allowed to proceed in darkness for 90 min.

For protein separation, the reduced and alkylated sample is added with .2 volume of 5x SDS gel sample buffer and boiled for 5 min. The cooled sample is then applied to a preparative SDS gel with the dimensions 20cm x 20cm x 1.5 mm. After electrophoresis, the gel is sliced perpendicular to the electrophoresis dimension into 10 strips of equal size. These strips represent 10 size bins for the intact proteins. The proteins in the gel strips are then subjected to in-gel digestion using standard protocols.

For peptide separation, the peptides that are extracted from the gel slices are subjected to three sequential chromatographic separations. First, they are separated by cation exchange chromatography. Second, the biotinylated peptides are isolated by avidin chromatography. Third, peptides are further fractionated by capillary reverse-phase chromatography.

For cation exchange chromatography, a cation exchange HPLC column is used (PolyLC Inc., Columbia MD; 2.1 mm x 20 cm, 5 µm particles, 300 Å pore size, Polysulfoethyl A strong cation exchange material). The following buffers are used: Buffer A, 10 mM KH₂PO₄, 25% CH₃CN, pH 3.0; Buffer B, 10 mM KH₂PO₄, 25% CH₃CN, 350 mM KCl, pH 3.0. The following gradient is run:

| Time (min) | %B |
|---|---|
| 0 | 0 |
| 30 | 25 |
| 50 | 100 |

The flow rate is 200 µL per minute. Fractions are collected at 1-2 minute intervals. Anywhere from about 200 microgram up to about 5 mg of digested, ICAT™ labeled total protein is loaded on this column, usually using a 2 mL sample loop. It is important to acidify the samples down to pH 3.0 or below before loading onto the cation exchange column because peptides will not be fully charged at higher pH values and can possibly not stick to the column. The gradient shown is designed to spread out the elution of doubly-charged peptides as much as possible, with these peptides usually eluting starting at about 8-9 minutes into the run until approximately minutes 15-16, after which triply charged peptides begin to elute. 30 fractions are collected over the duration of the gradient.

For avidin affinity chromatography, an Ultralink Monomeric Avidin (Pierce, cat # 53146) is used. A small piece of glass wool is packed into the neck of a glass pipette tube. 400 µl of avidin chromatography material is packed into the tube (slurry comes at 50% dilution, so 800 µl of 50% slurry is added in order to get 400 µl avidin chromatography material). The beads are allowed to settle, and the column is washed with 2 X PBS to bring the beads down off the side of the tube. The packed column is washed through with 30% acetonitrile (ACN) with 0.4% trifluoroacetic acid (TFA) until the flow-through pH changes to ∼1, and then another column volume of the ACN/TFA is washed through. This acidic step is to get rid of polymers associated with the beads. The column is washed with 2 X PBS pH 7.2 until the pH is -7.2. Thereafter, the column is washed through with 3 more column volumes (1200 µl) of same buffer.

The column is washed with 3-4 column volumes of biotin blocking buffer (2 mM d-biotin in PBS). This biotin blocks the more retentive avidin sites on the column, ensuring recovery of the sample from the remaining binding sites later on.

Loosely bound biotin is washed off with -6 column volumes (2,400 µl) of regeneration buffer (100 mM glycine, pH 2.8), until the flow-through pH changes to -2.8. This glycine solution is sterilized by autoclaving before use and stored at 4°C, where it will last one week.

The column is washed with 6 column volumes of 2 X PBS to return column to proper pH (∼7.2). The flow-through pH is monitored. The peptide samples consisting of individual or pooled ion exchange column fractions are then applied to the column and incubated in the column for -20 min.. Unbound material is then washed from the column by applying 5 column volumes of 2 X PBS, pH 7.2, and fractions are collected. The column is further washed through with 5 X column volumes of 1 X PBS (this step is to reduce the salt concentration), and 6 column volumes of 50 mM AMBIC (ammonium bicarbonate), pH 8.3, with 20 % methanol (MeOH) while continuing to collect fractions (50 mM AMBIC is to bring the salt concentration down; MeOH is to get rid of hydrophobic peptides). Biotinylated peptides are eluted with elution buffer (30% ACN / 0.4% TFA) and collected manually in a glass tube. These samples are further separated by capillary reverse-phase chromatography.

For reverse-phase chromatography, purified biotinylated peptides are separated by reverse-phase capillary chromatography using standard protocols. The solvent gradient is chosen so that the peptides elute over 60 min. If 1 min fractions are collected and analyzed in the mass spectrometer, 60 bins are created by this procedure.

For mass analysis and sequencing, biotinylated peptides eluting from the RP-columns are analyzed by ESI-MS/MS for the generation of the database and by ESI-MS for database searching. For the database construction, an ion trap mass spectrometer is used with a mass accuracy of approximately 1 mass unit. For the mass measurement for database searching, an ESI-TOF mass spectrometer is used with a mass accuracy exceeding 50 ppm and a resolution exceeding 10.000. That means that a peptide at 1000 mass units can be distinguished from a peptide at 1000.1 mass units. If a mass range for tryptic peptides is assumed to be between 500 and 3000 mass units, a mass spectrometer at that performance would generate 25,000 bins.

In the case of proteome analysis of the yeast *S*. *cerevisiae,* the genome of this organism contains approximately 6200 open reading frames (ORF's). The yeast proteome therefore is expected to be approximately 6200 different proteins, disregarding differentially modified forms of the same protein. Tryptic digestion of a yeast proteome would yield approximately 350,000 peptides if empirically derived specificity rules for trypsin are applied. This sample complexity is reduced to approximately 35,000 peptides if only the cysteine-containing peptides are extracted, based on the chemical derivatization with the ICAT™ type reagents. The total number of bins available from the procedure described above is 10x30x60x25,000= 4.5 x10⁸ and therefore by far exceeds the number of peptides expected from a total yeast proteome analysis. It is therefore expected that for a sample of the complexity of a yeast extract, the procedure for the generation of database search data can be simplified. As an example, the gel electrophoresis sizing step for proteins can be optionally eliminated.

Neither the procedure for the generation of the data to be entered into the database nor the procedure for the generation of data to search the database are fixed. Therefore, for optimization, depending on the degree of sample complexity, the number of bins available can be easily adjusted. Generally, the number of bins chosen for the generation of the database is high, whereas the number of bins for generating the database search data would be chosen as low as possible to maximize the sample throughput. The number of bins available can be easily adapted in various ways. Firstly, the inclusion of additional orthogonal separation dimensions can be considered for proteins or peptides. For protein separation, isoelectric focusing, ion exchange chromatography, hydroxylapatite chromatography, or similar electrophoretic or chromatographic techniques can be included. For peptide separation, separation based on peptide size or capillary electrophoresis methods can be included.

Secondly, the separation range for the separation methods described above can be extended. Protein sizing can be extended by using gradient gels or longer gels with extended separation range. For the chromatographic peptide separation methods, the number of bins can be easily expanded by generating extended, shallower gradients and/or by sampling more frequently. Finally the number of bins is dependent on the resolution and mass accuracy of the mass analyzer used. Adding a mass analyzer with higher performance will decrease the number of bins provided by the separation methods employed in the procedure.

### EXAMPLE III

### Use of ESI-TOF for MS Analysis of Complex Samples

This example describes a method for determining a polypeptide identification subindex using the mass of polypeptide fragments determined by ESI-TOF.

The masses of a set of polypeptides, or fragments thereof, were determined using ESI-TOF in order to constrain a polypeptide identification index by mass values to generate a polypeptide identification subindex for use with complex genomes. Two peptides, ASHLAGAR (Pl) and RPPGFSPR (P2), were infused together into an ESI-TOF. The mixture was intended to simulate co-elution of peptides during HPLC separation. Spectra were acquired at low (Figure 4A) and high (Figure 4B) V_{Nozzle-Skimmer}.

As can be seen in Figure 4B there are numerous fragment ions present for both peptides. No fragment ions appeared above the Pl 1 + ion and so this m/z range was omitted from Figure 4B. An algorithm was written to deconvolute the mixture of parent and fragment ions in a single mass spectrum (Figure 4B). P1 and P2 sequences were placed into a list of all possible tryptic peptides from the database of 60,884 human polypeptides. Comparison of P1 and P2 observed masses (Figure 4A) to all possible tryptic peptides from the 60,884 polypeptides, produced a list of 57 and 124 isobars, respectively. The list of b- and y-ion fragments from all isobars calculated to 10 ppm was then compared to the observed fragment ions between 500 and 825 m/z in Figure 4B. This process produced a list of 12 and 13 possible polypeptide identifications for P1 and P2, respectively, as shown in Figure 4C. This method is useful for applying a constraint to a polypeptide identification index, for example, to generate a polypeptide identification subindex for use with complex genomes. In-source CID on peptides can be acquired easily and quickly in a continuously alternating fashion by ESI-TOF and potentially by in-source decomposition in MALDI-TOF. Furthermore, the method does not result in loss of data as occurs with tandem MS where, during CID of a selected ion, co-eluting ions are necessarily omitted from analysis. If the peptides are of low abundance (that is, low signal-to-noise) then they will not be selected for CID by data dependent (DD) tandem MS processes. This is because standard DD processes examine first the base peak (that is, most intense ion per m/z window), carries out CID on it, dynamically excludes it from further consideration, and proceeds to the next most abundant ion.

The peptides shown in Figure 4C represent a reduction in complexity from over 60,000 possible polypeptides to about 12 or 13 polypeptides. Additional characteristics are determined, for example, atomic mass, amino acid composition, partial amino acid sequence, apparent molecular weight, pI, and order of elution on specific chromatographic media under defined conditions, and the like, for parent polypeptides or one or more additional peptide fragments thereof. Methods for fractionating polypeptides have been previously described (see, for example, Scopes, Protein Purification: Principles and Practice, 3rd ed.,Springer Verlag, New York (1993)). A sufficient number of characteristics are determined so that a single polypeptide in the polypeptide identification index is identified.

### EXAMPLE IV

### Shotgun Peptide Sequencing for Generating a Polypeptide Identification Index

This example describes the sequencing of peptides using high mass accuracy.

A method was developed for sequencing peptides using high mass accuracy (50 ppm or better) parent and fragment ions in an ESI-TOF LC/MS system. The method involved introducing purified protein digest samples at alternately high or low ion source gradient potentials, wherein fragmentation data from peptides can be recorded and combined with non-fragmentary data for positive identification of peptide sequence and parent protein through database searching. Although cysteine content was used in this particular example, it is not required that cysteine content be a determined characteristic. In contrast to traditional tandem MS, all peptides entering the MS together were fragmented without selection of an individual ion. All fragment ions for the two peptides simultaneously fragmented were measured together, in contrast to tandem MS, where fragment ions are measured for a single peptide at a time.

Briefly, *Saccharomyces cerevisiae* (BWG1-7A) was grown in YPD media to an OD₆₀₀ of 3 (just past log phase). Spheroplasts were created by lyticase digestion and subsequently mechanically lysed. Nuclei were isolated by centrifugation (13,000 rpm for 30 min) and lysed by addition of ammonium sulfate. Insoluble material was spun out (28,000 rpm for 90 min) and supernatant extracted. Proteins were precipitated with ammonium sulfate. The precipitate was resuspended and dialyzed against 20 mM Hepes, 20% glycerol, 10 mM MgSO₄, 1 mM EGTA, and 75 mM ammonium sulfate.

Protein extract (5.6 mg) was dried in a Speedvac (Savant Instruments; Holbrook NY) and resuspended and denatured by boiling in 0.5% SDS, 50 mM Tris, 1 mM EDTA for 3 min. Dithiothreitol (DTT; 5mM) was added, and the sample was incubated at 50°C for 30 min. Proteins were precipitated in cold acetone/ethanol (EtOH) (50:50 v:v). The pellets were washed and resuspended in 0.05% SDS, 6 M urea, 50 mM Tris, pH 8.3, and 1 mM EDTA, and 3 mg EZ-Link™ PEO-Iodoacetyl Biotin (Pierce catalog # 21334; Pierce Chemical Co.; Rockford IL) was added. The sample was incubated in the dark with agitation for 90 min. An equal volume of 20 mM DTT was added to quench the biotinylation reaction. The sample was then diluted 6-fold by volume with 50 mM Tris, 1 mM EDTA. 200 µg trypsin was added, and the sample was incubated at 37°C overnight.

Approximately 2 mg of trypsin-digested solution was run over a PolysulfoethylA strong cation exchange column (Poly LC #202SE0503, 2.1 mm x 200 mm, 300 A, 5µm) (gradient = 0% to 25% over 30 min, 25% to 100% over 20 min, 300 mM KCl in 10 mM K₂PO₄, 25% Acetonitrile (ACN), pH 3.0, at 200 µl/min) with fractions collected every 5 minutes. Each fraction was purified for biotinylated cysteinyl peptides using an ABI affinity cartridge, cartridge holder, and attendant buffer system (ABI #4326740 and #4326688-90; Applied Biosystems; Foster City CA). Purified fraction volumes (representing 0.1 cation exchange absorbance units from 214 nm UV trace) were dried to completion in a Speedvac and resuspended in 0.2% acetic acid (HOAc) to 5 µl volume.

The dried sample was separated on reverse phase by means of an LC Packings (San Francisco CA) integrated autosampler/µHPLC system coupled to a 100 µm x 10 cm fused silica handpacked C-18 (Michrom Magic C18) column using a linear gradient of ACN (solvent B) from 5% to 50% at approximately 700 nl per minute over 60 minutes. Solvent A was 0.2% HOAc/0.001% heptafluorobutyric acid. The fused silica capillary column was coupled to a 75 to 30 µm taper tip fused silica needle (New Objective, FS360-75-30-N) through a stainless steel 0.15 mm bore microvolume Valco union (MU1XCS6) to which 4000 VDC (voltage direct current) was applied. The biotinylated peptides were analyzed twice by µLC/MS using an ABI Mariner Biospectrometry Workstation ESI-TOF mass spectrometer, first using a nozzle/skimmer (V_{NZ}) potential of 70/25 VDC, then re-run using a V_{NZ} potential of 200/25 VDC.

A list of non-fragmented potential peptides was developed through manual examination of the entire chromatographic run. Identifiable doubly, triply, and quadruple charged m/z monoisotopic values were placed into a spreadsheet and deconvoluted. Deconvoluted masses were supplied to a simple database searching program that gave a list of peptides fitting the following criteria: mass with precision of 30 ppm, peptide containing the presence of a cysteinyl residue, peptide containing tryptic sites at both ends, two possible tryptic cleavage sites are allowed to be missing, cysteine modified by 414.1931 Da (PEO-iodoacetylation). Each potential peptide sequence was then fragmented *in silico* to develop a list of possible fragment ion m/z values. Fragmented chromatographic run was then manually inspected for the presence or absence of those potential fragments that would lie within the experimentally set m/z range. Each potential peptide candidate was then evaluated as to its match with the actual fragments from the mass spectrometry data to within 40 ppm error. Further, the fragments and precursor masses they came from were manually entered into SEQUEST™ and searched against the same database to develop cross correlation (XCorr) and delta correlation (dCn) scores for comparisons to ion trap data. Finally, the same sample was run on a ThermoFinnigan LCQ ion trap LC/MS(MS) setup using a similar reverse phase separation scheme. The results were submitted for automated database analysis using SEQUEST™ (Eng et al., *supra,* 1994) to verify the ESI-TOF results.

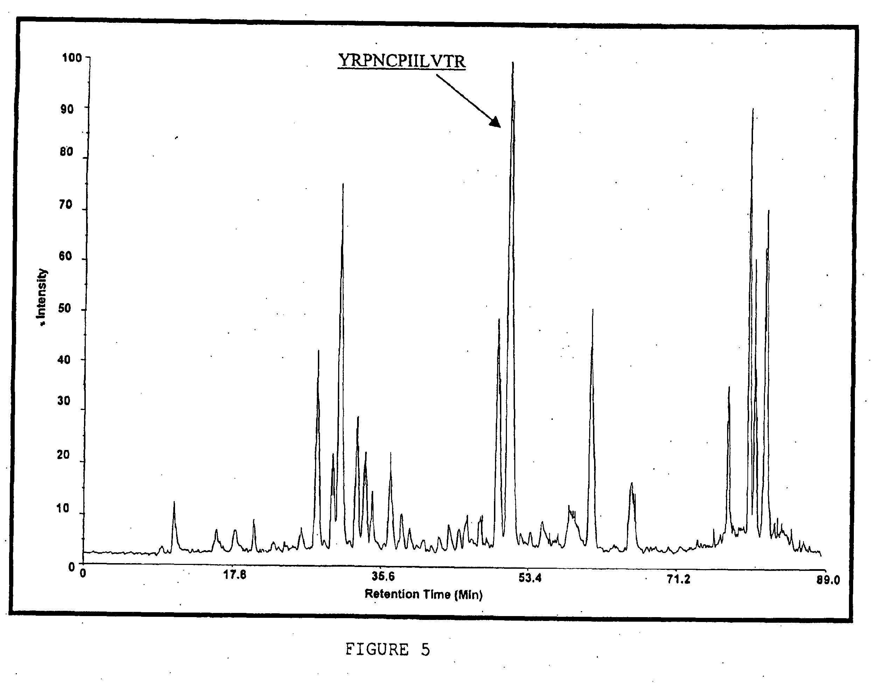

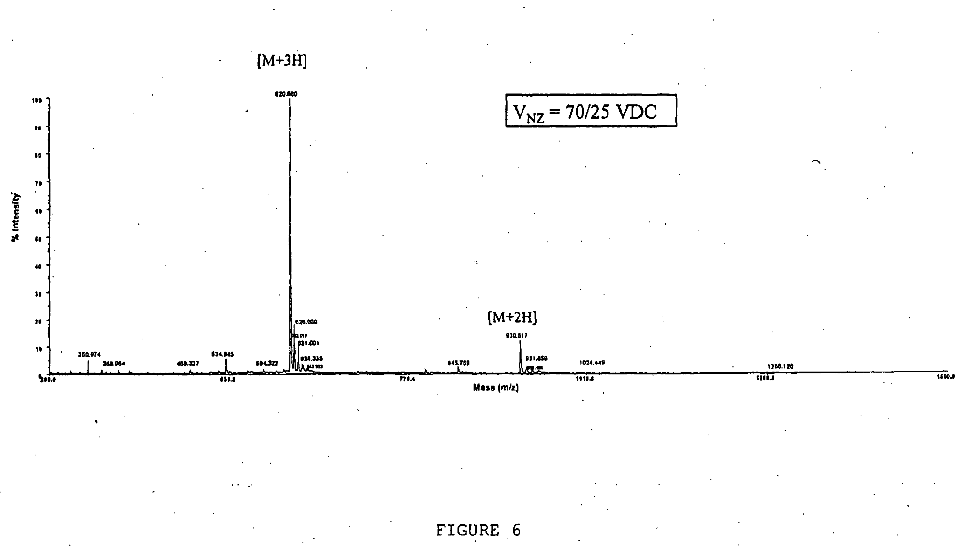

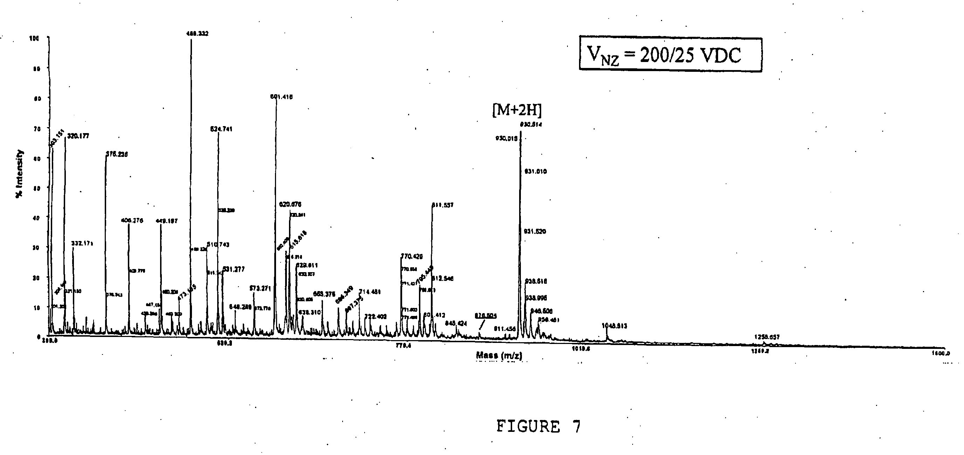

The scans from the chromatographic region indicated in Figure 5 were summed for both the non fragmentary and fragmentary runs and are seen in Figures 6 and 7, respectively. The triply charged peak at 620.3436 m/z and the doubly charged peak at 930.0258 m/z were deconvoluted to a [M+H] = 1859.03. The yeast open reading frame database was searched for cysteine containing peptides at this [M+H] that were fully tryptic, containing two potentially missed cleavages, and within 30 ppm of the deconvoluted mass. In Figure 5, the arrow points to peptide YRPNCPIILVTR (SEQ ID NO:26), which was resolved as a single peptide on chromatography. Although no ion selection was performed during MS, the peptide was still positively identified. The results are shown in Table 1, where one peptide, sequence YRPNCPIILVTR (SEQ ID NO:26), was positively identified by 22 out of 33 ions being seen (Table 1, SEQ ID NOS:27-33, 26 and 34-38, respectively).

**TABLE 1**

| Possible peptides matching [M+H] = 1859.03 @ 30.0 ppm mass accuracy & 414.1931 Da. added to Cysteine (PEO linked)^{(a)(b)} | | | |
|---|---|---|---|
| Sequence | Theoretical [M+H] | Error from Theoretical | # of fragment ions matched to number of possible fragment ions within experimental m/z range of instrument (within 40 ppm error) |
| LDNCVINEKGIVK | 1858.976440 | 28.8 ppm | 3/32 |
| RPMICLKNNSLR | 1858.981201 | 26.3 ppm | 3/30 |
| NKANICLAKNDLK | 1858.991699 | 22.8 ppm | 3/32 |
| YLIVLTQNCHLK | 1858.991699 | 20.6 ppm | 1/30 |
| FYTKRLCTVSVK | 1858.991699 | 20.6 ppm | 0/29 |
| MLRNLVVRNACR | 1858.992432 | 20.2 ppm | 0/35 |
| GCEVWSGKLRAAR | 1858.998901 | 16.7 ppm | 3/40 |
| YRPNCPIILVTR | 1859.003052 | 14.5 ppm | 22/31 (XCorr: 3.24, dCn: 0.6) |
| SIQLTLKGPSGCLK | 1859.012939 | 9.2 ppm | 0/38 |
| VITLEKICEVAAR | 1859.012939 | 9.2 ppm | 1/30 |
| IDRLLLKCDLSR | 1859.024048 | 3.2 ppm | 1/30 |
| RGTDVLKALCLGAK | 1859.024048 | 3.2 ppm | 0/34 |
| VKPIRGFCQRLK | 1859.050659 | 11.1 ppm | 0/28 |

| | | | |
|---|---|---|---|
| (a) from Yeast open reading frame database (b) EZ-Link™ PEO-Iodoacetyl Biotin, Pierce # 21334 | | | |

**TABLE 2**

| Matched fragment ions (*) within experimental m/z range of instrument & ppm error of measured m/z value | | | | | | |
|---|---|---|---|---|---|---|
| Seq. | # | b (+1) | b (+2) | Y (+1) | Y (+2) | # |
| Y | 1 | ---- | - ----- | ----- | 930.0054 | 12 |
| R | 2 | 320.1722 (36.9 ppm)* | ----- | ----- | 848.4738 (8.8 ppm)* | 11 |
| P | 3 | 417.2250 (20.9 ppm)* | ---- | ----- | 770.4232 (7.3 ppm)* | 10 |
| N | 4 | 531.2679 (17.9 ppm)* | ----- | 1442.7858 | 721.8968 (7.2 ppm)* | 9 |
| C | 5 | 1048.4702 (0.7 ppm)* | 524.7390 (5.0 ppm)* | 1328.7429 | 664.8754 (4.7 ppm)* | 8 |
| P | 6 | 1145.5229 | 573.2654 (37.7 ppm)* | 811.5406 (8.9 ppm)* | 406.2742 (3.9 ppm)* | 7 |
| I | 7 | 1258.6071 (21.0 ppm)* | 629.8074 (7.1 ppm)* | 714.4879 (13.2 ppm)* | 357.7478 | 6 |
| I | 8 | 1371.6912 (3.4 ppm)* | 686.3496 (0.9 ppm)* | 601.4037 (5.3 ppm)* | 301.2058 | 5 |
| L | 9 | 1484.7753 | 742.8915 (14.5 ppm)* | 488.3196 (23.5 ppm)* | --- | 4 |
| V | 10 | ---- | 792.4257 | 375.2356 (1.3 ppm)* | ---- | 3 |
| T | 11 | ---- | 842.9496 | ---- | ----- | 2 |
| R | 12 | ---- | 921.0001 | ----- | ----- | 1 |

Table 2 shows the theoretical masses for each fragment that lies within the experimental m/z range (300-1500 m/z), with matching peaks indicated with an "*", with their attendant ppm error. The masses were entered manually into SEQUEST™ to develop the cross correlation and delta correlation scores shown in Table 1. Finally, this peptide was also found by SEQUEST™ when searching ion trap data from the same sample, with XCorr of 3.75 and delta correlation score of 0.325.

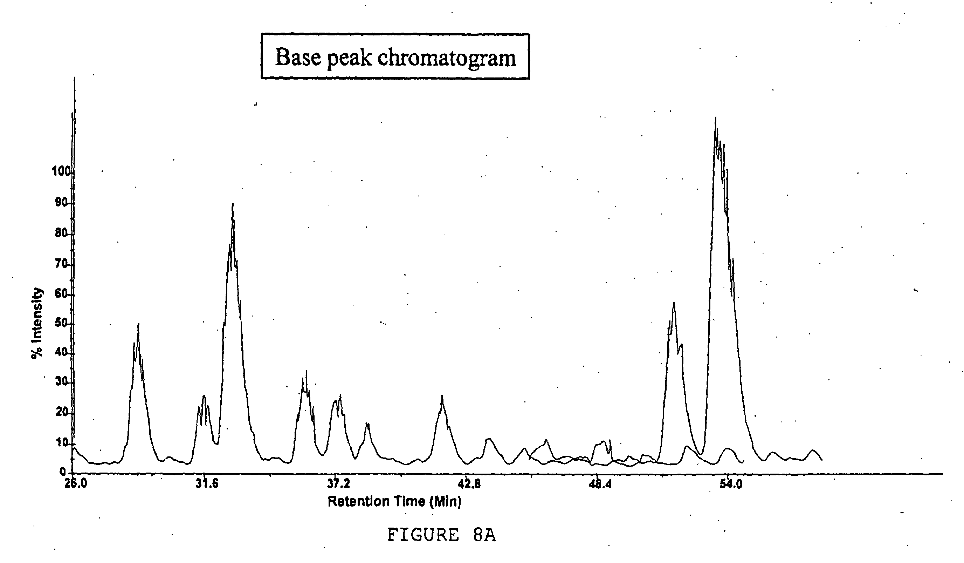

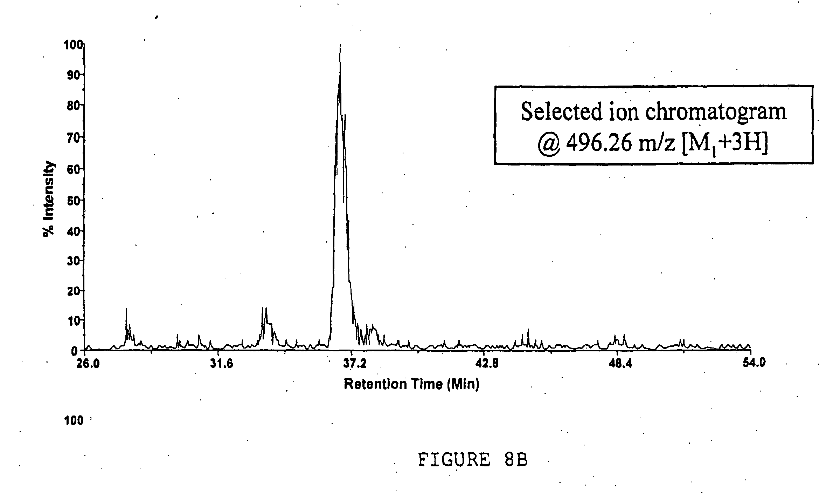

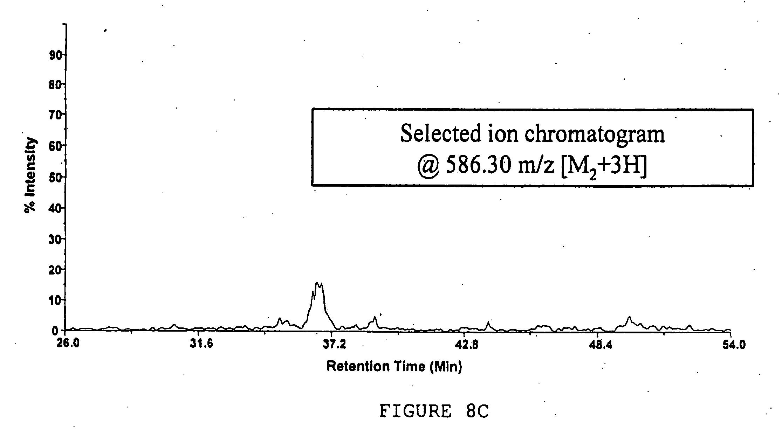

In a more complex sample, two peptides co-eluted in the chromatogram (see Figure 8). The co-eluting peptides were fragmented in the source region without ion selection. Although MS was performed without ion selection, the two co-eluting peptides were identified.

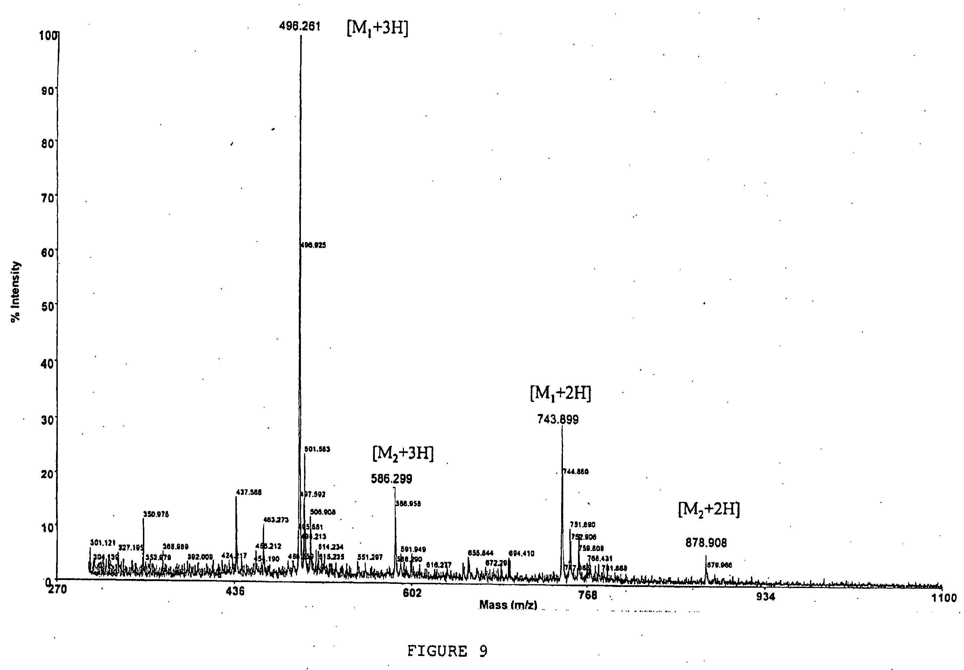

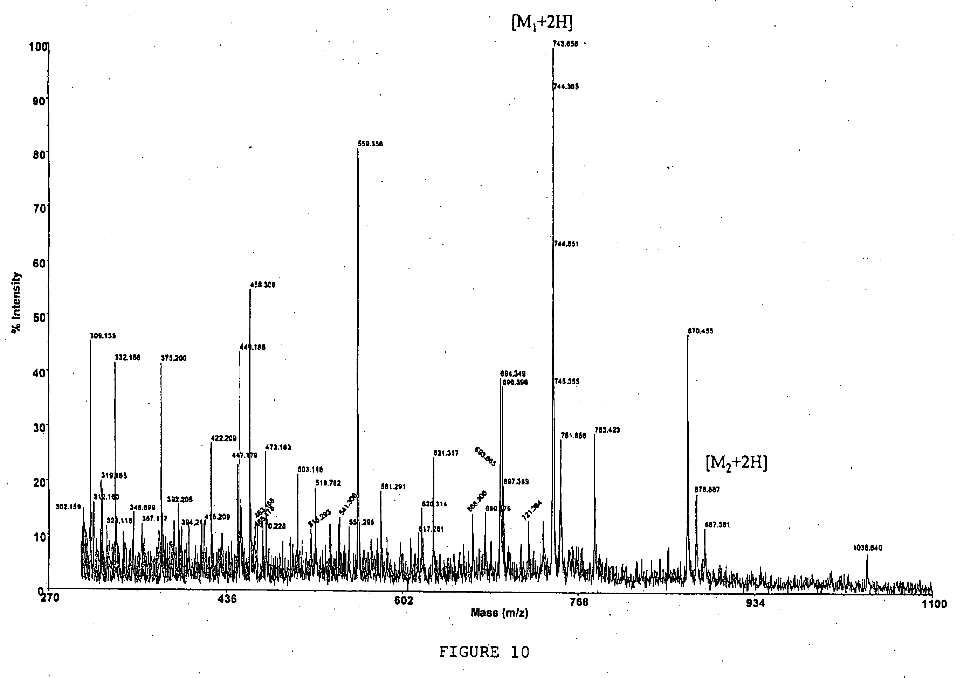

The non fragmentary masses, as shown in Figure 9, were entered into the database searching program used above. The potential peptides were fragmented *in silico,* and the resulting fragment masses compared to the fragmentary spectrum m/z values seen in Figure 10. Table 3 shows the candidate peptides for [M1+H] = 1486.76 and the number of ions matched. The peptide sequence was determined to be VCSSHTGLIR (SEQ ID NO:44) (Table 3, SEQ ID NOS:39-49, respectively).

**TABLE 3**

| Possible peptides matching [M₁+H] = 1486.76 @ 30.0 ppm mass accuracy & 414.1931 Da. added to Cysteine (PE0 linked)^{(a)(b)} | | | |
|---|---|---|---|
| Sequence | Theoretical [M+H] | Error from Theoretical | # of fragment ions matched to number of possible fragment ions within experimental m/z range of instrument (within 40 ppm error) |
| KHCQSQVSR | 1486.725342 | 23.3 ppm | 0/25 |
| MMIAFTCKK | 1486.728760 | 21.0 ppm | 1/25 |
| EFRFCRSK | 1486.729248 | 20.7 ppm | 0/21 |
| KTLMSVCYK | 1486.746582 | 9.0 ppm | 1/26 |
| KCKNGPSPNK | 1486.750366 | 6.5 ppm | 0/31 |
| VCSSHTGLIR | 1486.750366 | 6.5 ppm | 9/31 (XCoff:1.5 dCn:0.708) |
| CYLQYRVK | 1486.754517 | 3.7 ppm | 0/29 |
| LTVQRPQCK | 1486.786743 | 18.0 ppm | 3/27 |
| VVQLQNICR | 1486.786743 | 18.0 ppm | 2/27 |
| FIHVPTCKK | 1486.790771 | 20.7 ppm | 1/25 |
| TIGLLLCDPK | 1486.800659 | 27.3 ppm | 0/27 |

| | | | |
|---|---|---|---|
| (a) from Yeast open reading frame database (b) EZ-Link™ PEO-lodoacetyl Biotin, Pierce # 21334 | | | |

**TABLE 4**

| Possible peptides matching [M₂+H] = 1756.84 @ 30.0 ppm mass accuracy & 414.1931 Da. added to Cysteine (PE0 linked)^{(a)(b)} | | | |
|---|---|---|---|
| Sequence | Theoretical [M+H] | Error from Theoretical | # of fragment ions matched to number of possible fragment ions within experimental m/z range of instrument (within 40 ppm error) |
| FYRCYNSYAR | 1756.793457 | 26.5 ppm | 0/25 |
| YKDCEDWKQK | 1756.803345 | 20.8 ppm | 0/25 |
| FTDMQLELCSR | 1756.806641 | 19.0 ppm | 0/29 |
| MSCESEQFLLR | 1756.806641 | 19.0 ppm | 0/29 |
| ETYEIICDQTK | 1756.813232 | 15.2 ppm | 1/26 |
| MATDERCIFR | 1756.817871 | 12.6 ppm | 0/28 |
| SYSSCPAVSQSVK | 1756.824341 | 16.7 ppm | 0/33 |
| TIDFKFPECDK | 1756.828369 | 6.6 ppm | 0/29 |
| NAPGDCVSPVQQK | 1756.835571 | 2.5 ppm | 0/33 |
| TLACYSDAIVMR | 1756.843018 | 1.7 ppm | 1/31 |
| GHMPCTSTISGR | 1756.846924 | 4.0 ppm | 6/32 (XCorr: 1.1 dCn: 0.751) |
| DGVRYVIFDCR | 1756.850952 | 6.3 ppm | 0/29 |
| MFPIGCATFLSR | 1756.858276 | 10.4 ppm | 1/31 |
| LKGKDSFSMVCK | 1756.879395 | 22.4 ppm | 0/33 |

| | | | |
|---|---|---|---|
| (a) from Yeast open reading frame database (b) EZ-Link™ PEO-Iodoacetyl Biotin, Pierce #21334 | | | |

Table 4 shows the candidate peptides for [M2+H] = 1758.84 and the number of ions matched. The peptide sequence was determined to be GHNIPCTSTISGR (SEQ ID NO:60) (Table 4, SEQ ID NOS:50-63, respectively).

In the case of both peptides ([M1+H] = 1486.76 and [M2+H] = 1758.84), the fragment m/z values were manually entered into SEQUEST™ and XCorr and dCn values developed. They are listed in Tables 3 and 4, respectively.

Tables 5 and 6 show which fragment ions were matched ("*"), the theoretical m/z of the fragments, and the ppm error of the actual m/z values. Finally, these peptides were also found by SEQUEST™ when searching ion trap data from the same sample, with XCorr of 2.84 and delta correlation score of 0.198 for [M1+H] = 1486.76, and XCorr of 3.18 and delta correlation score of 0.299 for [M2+H] = 1758.84.

**TABLE 5**

| Matched fragment ions (*) within experimental m/z range of instrument & ppm error of measured m/z value | | | | | | |
|---|---|---|---|---|---|---|
| Seq. | # | b (+1) | b (+2) | Y (+1) | Y (+2) | # |
| V | 1 | ---- | ----- | 1468.7506 | 743.8792 | 10 |
| C | 2 | 617.2786 | 309.1432 | 1387.6821 | 694.3450 (34.6 ppm)* | 9 |
| S | 3 | 704.3106 | 352.6592 | 870.4798 (8.3 ppm)* | 435.7438 (1.4 ppm)* | 8 |
| S | 4 | 791.3427 | 396.1752 | 783.4478 (12.0 ppm)* | 392.2278 (11.5 ppm)* | 7 |
| H | 5 | 928.4016 | 464.7047 | 696.4158 (16.5 ppm)* | 348.7118 (5.7 ppm)* | 6 |
| T | 6 | 1029.4492 | 515.2285 | 559.3569 (0.9 ppm)* | ----- | 5 |
| G | 7 | 1086.4707 | 543.7393 | 458.3091 (12.9 ppm)* | ----- | 4 |
| L | 8 | 1199.5548 | 600.2813 | 401.2876 | ----- | 3 |
| I | 9 | 1312.6389 | 656.8234 | --- | ----- | 2 |
| R | 10 | 1468.7400 | 734.8739 | | --- ----- | 1 |

**TABLE 6**

| Matched fragment ions (*) within experimental m/z range of instrument & ppm error of measured m/z value | | | | | | |
|---|---|---|---|---|---|---|
| Seq. | # | b (+1) | b (+2) | Y (+1) | Y (+2) | # |
| G | 1 | ----- | ----- | ----- | 878.9274 | 13 |
| H | 2 | ----- | ----- | ----- | 850.4166 | 12 |
| N | 3 | 309.1311 (30.7 ppm)* | ----- | ----- | 781.8872 | 11 |
| I | 4 | 422.2152 (5.0 ppm)* | ----- | 1448.7235 | 724.8657 | 10 |
| P | 5 | 519.2679 | ----- | 1335.6395 | 668.3237 (20.6 ppm)* | 9 |
| C | 6 | 1036.4702 | 518.7390 | 1238.5868 | 619.7973 | 8 |
| T | 7 | 1137.5179 | 569.2629 | 721.3845 (14.6 ppm)* | 361.1961 | 7 |
| S | 8 | 1224.5499 | 612.7789 | 620.3368 | 310.6723 | 6 |
| T | 9 | 1325.5977 | 663.3027 | 533.3047 | ----- | 5 |
| I | 10 | 1438.6818 | 719.8448 | 432.2570 (17.4 ppm)* | ----- | 4 |
| S | 11 | ----- | 763.3608 | 319.1730 (18.5 ppm)* | ----- | 3 |
| G | 12 | ----- | 791.8715 | ---- | ----- | 2 |
| R | 13 | ----- | 869.9221 | ----- | ----- | 1 |

These results show that positive identifications of peptide sequences were obtained for peptides in pure form or concurrently with other peptides, that is, singly and multiply eluting peptides, respectively. Co-eluting peptides were "shotgun" sequenced using in-source CID on a single-stage ESI-TOF mass spectrometer. Relative to traditional peptide sequencing via tandem MS, where parent ions are selected sequentially, a combination of cysteine-constraint, high mass accuracy and high mass resolution allowed peptides to be shotgun sequenced in parallel. The method can also be used with ICAT (Gygi et al., Nature Biotechnol. 17:994-999 (1999)) for the analysis of ICAT-labeled peptides in one analysis.

These results demonstrate that parent proteins can be identified from a combination of determined characteristics. In this example, the determined characterstics were (1) cysteine content, (2) accurate peptide mass and (3) accurate peptide fragment mass without selection of a specific ion.

### EXAMPLE V

### Generation of a Drosophila Database as a Polypeptide Identification Index

This example describes the generation of a database for identifying *Drosophila* proteins.

*Drosophila* was chosen as a model organism representative of a species for which the complete genomic sequence has been determined and for which the genome sequence contains a large number of exons. The purpose of the experiment was the generation of a large set of peptides and proteins from a species for which the complete genomic sequence has been determined, to separate the peptides by multi dimensional chromatography, to identify the peptides via the generation of CID spectra and sequence database searching, and to annotate each identified peptide with a bar code of information obtained during the fractionation/identification process.

Schneider (S2) cells (10⁹) from *Drosophila melanogaster* were prepared by culturing in suitable tissue culture medium. The cells were lysed, and the resulting lysates were fractionated by differential centrifugation into a nuclear fraction, a cytoplasmic fraction and a microsomal fraction. Each fraction was split into two identical halves, and the proteins contained in each sample were labeled with either the isotopically normal (d0) or the isotopically heavy (d8) ICAT reagents. The labeled samples representing the same subcellular fraction were then combined to generate a protein mixture of 1:1 isotopically labeled proteins and trypsinized. The resulting peptides were fractionated by cation exchange chromatography as described in Example II, followed by avidin chromatography for the selective isolation of the tagged peptides, and reverse phase chromatography. The separated peptides were analyzed and subjected to CID in an LCQ ion trap mass spectrometer. The resulting peptide CID spectra were subjected to sequence database searching using the fly sequence database from the fly genome sequencing consortium and the Sequest™ search algorithm.

To identify the proteins by sequence database searching using peptide CID spectra, the following search parameters were used: Double tryptic peptides containing minimally one cysteine residue were considered only. A delta correlation factor bigger than 0.1 was required and an X-correlation coefficient bigger than 2.0 was required for a doubly charged precursor ion (X-corr. >1.5 for singly charged peptides; X-corr. >2.5 for triply charged peptides).

For the nuclear fraction, 1470 proteins were identified. For the cytoplasmic fraction, 967 proteins were identified. For the microsomal fraction, in excess of 1500 proteins were identified. Therefore, in this analysis, about 4000 proteins were identified. These data therefore support the use of multidimensional chromatography, tandem mass spectrometry and sequence database searching for establishing an annotated peptide database for a specific tissue, cell type or species.

These results demonstrate that a polypeptide identification index can be generated that can be used for the identification of a large number of polypeptides.

### EXAMPLE VI

### Use of Isoelectric Point as a Discriminating Characteristic for Polypeptide Identification

This example describes the use of isoelectric point (pI) as a characteristic for identification of a polypeptide.

The isoelectric point of peptides was tested as a discriminating component useful for a peptide bar code. The advantages of using the pI as a component of the bar code includes: i) the pI is easily and precisely predictable from the amino acid sequence of a peptide; ii) the pI can be precisely measured by IEF; and iii) the peptides can be efficiently recovered from the IEF gels.

An ion exchange fraction of the fly peptide samples described in Example V was further separated by isoelectric focusing in a polyacrylamide gel with immobilized pH gradient (IPG-IEF). The peptides focusing to a segment around pI 6.8-7.0 was cut out, and the peptides contained in the fraction were extracted and analyzed by LC-MS/MS. The resulting CID spectra were subjected to sequence database searching, as described in Example V, and the pI of each identified peptide was also calculated using the sequence of the best sequence database match. Table 7 shows a partial list of the results (SEQ ID NOS:64-117) as illustrative of the method. The pI of most identified peptides clustered around a narrow pI range, indicating that the pI can be precisely calculated from the sequence.

The data furthermore showed that there were a (low) number of peptides for which the best match resulted in a pI outside the range extracted from the gel. Closer examination of these peptides suggested that the sequence database match was almost certainly wrong for these peptides outside the pI range of the extracted gel region because the search scores were marginal, the peptides usually contained a high proportion of missed

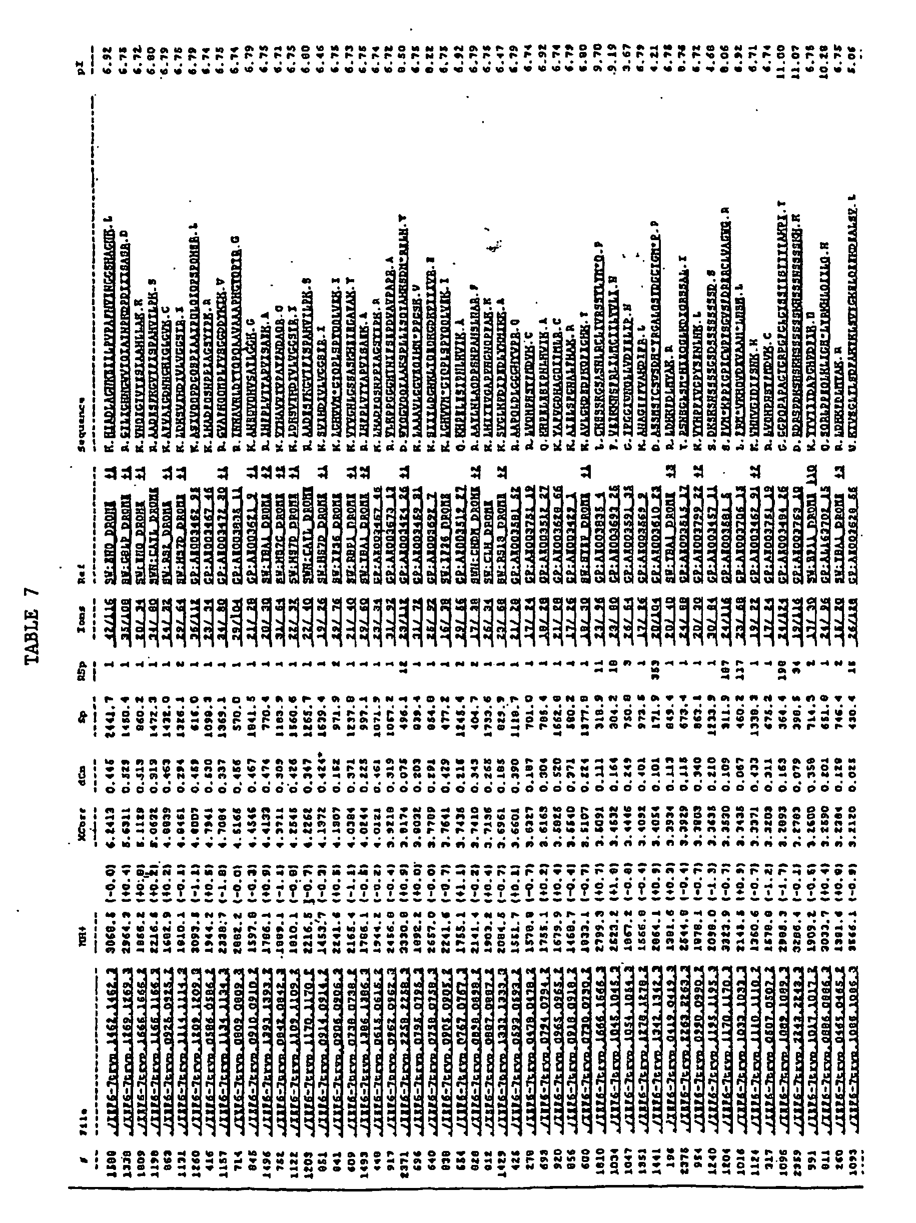

tryptic cleavage sites, and many of the peptides were non tryptic. Collectively, these data indicate the power of using the peptide pI as a component of the bar code for identifying a polypeptide from a polypeptide identification index.

These results show that pI can be used as a characteristic of a polypeptide for identifying a polypeptide using a polypeptide identification index.

## Claims

1. A method of identifying a polypeptide comprising:
fractionating polypeptide fragments by chromatography; and
subjecting a chromatographic fraction to mass spectrometry;
**characterised in that** said method further comprises:
determining two characteristics associated with a fragment of a polypeptide, one of said characteristics being the mass of said fragment of said polypeptide, said fragment mass being determined by mass spectrometry and one of said characteristics being the order of elution on a chromatographic medium;
comparing said characteristics to an annotated polypeptide index;
identifying one or more polypeptides in said annotated polypeptide index having said characteristics; and
quantitating the amount of said identified polypeptide in a sample containing said polypeptide.

2. A method as claimed in claim 1, wherein said fragment mass is determined at an accuracy in ppm of 1 ppm or greater ppm.

3. A method as claimed in claim 1, wherein said fragment mass is determined at an accuracy in ppm of 2.5 ppm or greater ppm.

4. A method as claimed in claim 1, wherein said fragment mass is determined at an accuracy in ppm of 5 ppm or greater ppm.

5. A method as claimed in claim 1, wherein said fragment mass is determined at an accuracy in ppm of 10 ppm or greater ppm.

6. A method as claimed in claim 1, wherein said fragment mass is determined at an accuracy in ppm of 100 ppm or greater ppm.

7. A method as claimed in any preceding claim, wherein said step of fractionating comprises fractionating by liquid chromatography.

8. A method as claimed in claim 7, wherein said step of fractionating comprises fractionating by reverse phase liquid chromatography.

9. A method as claimed in any preceding claim, wherein said step of quantitating comprises spiking said sample with an internal standard.

10. A method as claimed in any preceding claim, wherein said annotated polypeptide index comprises at least one empirically determined characteristic for each of the polypeptides in the index.

## Patentansprüche

1. Ein Verfahren zur Identifizierung eines Polypeptids, Schritte umfassend, bei denen man:
Polypeptidfragmente durch Chromatographie fraktioniert; und
eine chromatographische Fraktion einer Massenspektrometrie unterwirft;
**dadurch gekennzeichnet, dass** das Verfahren ferner Schritte umfasst, bei denen man:
zwei Charakteristika bestimmt, die mit einem Fragment eines Polypeptids assoziiert sind, wobei ein Charakteristikum die Masse des Fragments des Polypeptids ist, wobei die Masse des Fragments durch Massenspektrometrie bestimmt wird, und eins der Charakteristika die Reihenfolge der Elution von einem chromatographischen Medium ist;
die Charakteristika mit einem kommentierten Polypeptidindex vergleicht;
ein oder mehrere Polypeptide mit den Charakteristika in dem kommentierten Polypeptidindex identifiziert; und
die Menge des identifizierten Polypeptids in einer Probe, welche das Polypeptid enthält, quantifiziert.

2. Ein Verfahren, wie in Anspruch 1 beansprucht, **dadurch gekennzeichnet, dass** die Masse des Fragments mit einer Genauigkeit in ppm von 1 ppm oder mehr ppm bestimmt wird.

3. Ein Verfahren, wie in Anspruch 1 beansprucht, **dadurch gekennzeichnet, dass** die Masse des Fragments mit einer Genauigkeit in ppm von 2,5 ppm oder mehr ppm bestimmt wird.

4. Ein Verfahren, wie in Anspruch 1 beansprucht, **dadurch gekennzeichnet, dass** die Masse des Fragments mit einer Genauigkeit in ppm von 5 ppm oder mehr ppm bestimmt wird.

5. Ein Verfahren, wie in Anspruch 1 beansprucht, **dadurch gekennzeichnet, dass** die Masse des Fragments mit einer Genauigkeit in ppm von 10 ppm oder mehr ppm bestimmt wird.

6. Ein Verfahren, wie in Anspruch 1 beansprucht, **dadurch gekennzeichnet, dass** die Masse des Fragments mit einer Genauigkeit in ppm von 100 ppm oder mehr ppm bestimmt wird.

7. Ein Verfahren, wie in irgendeinem vorhergehenden Anspruch beansprucht, **dadurch gekennzeichnet, dass** der Schritt der Fraktionierung eine Fraktionierung durch Flüssig-Chromatographie (liquid chromatography) umfasst.

8. Ein Verfahren, wie in Anspruch 7 beansprucht, **dadurch gekennzeichnet, dass** der Schritt der Fraktionierung eine Fraktionierung durch Reverse Phase Flüssig-Chromatographie (reverse phase liquid chromatography) umfasst.

9. Ein Verfahren, wie in irgendeinem vorhergehenden Anspruch beansprucht, **dadurch gekennzeichnet, dass** der Schritt des Quantifizierens es umfasst, die Probe mit einem internen Standard zu versetzen.

10. Ein Verfahren, wie in irgendeinem vorhergehenden Anspruch beansprucht, **dadurch gekennzeichnet, dass** der kommentierte Polypeptidindex mindestens ein empirisch bestimmtes Charakteristikum für jedes der Polypeptide in dem Index umfasst.

## Revendications

1. Procédé d'identification d'un polypeptide, comprenant les étapes suivantes :
- séparer des fragments du polypeptide par fractionnement chromatographique ;
- et soumettre les fractions chromatographiques à une analyse par spectrométrie de masse ;
lequel procédé est **caractérisé en ce qu'**il comporte en outre les étapes suivantes :
- déterminer deux caractéristiques associées à un fragment d'un polypeptide, dont l'une est la masse dudit fragment dudit polypeptide, masse qui est déterminée par spectrométrie de masse, l'autre de ces caractéristiques étant l'ordre d'élution sur un milieu chromatographique ;
- comparer ces caractéristiques avec celles qui figurent dans un index de polypeptides et de données concernant ceux-ci ;
- identifier, dans cet index de polypeptides, un ou plusieurs polypeptides qui possèdent lesdites caractéristiques ;
- et déterminer la quantité dudit polypeptide identifié dans un échantillon contenant ledit polypeptide.

2. Procédé conforme à la revendication 1, dans lequel ladite masse de fragment est déterminée avec une précision en ppm de 1 ppm ou plus.

3. Procédé conforme à la revendication 1, dans lequel ladite masse de fragment est déterminée avec une précision en ppm de 2,5 ppm ou plus.

4. Procédé conforme à la revendication 1, dans lequel ladite masse de fragment est déterminée avec une précision en ppm de 5 ppm ou plus.

5. Procédé conforme à la revendication 1, dans lequel ladite masse de fragment est déterminée avec une précision en ppm de 10 ppm ou plus.

6. Procédé conforme à la revendication 1, dans lequel ladite masse de fragment est déterminée avec une précision en ppm de 100 ppm ou plus.

7. Procédé conforme à l'une des revendications précédentes, dans lequel ladite étape de séparation par fractionnement comprend un fractionnement par chromatographie en phase liquide.

8. Procédé conforme à la revendication 7, dans lequel ladite étape de séparation par fractionnement comprend un fractionnement par chromatographie en phase liquide à polarité de phases inversée.

9. Procédé conforme à l'une des revendications précédentes, dans lequel ladite étape de détermination de quantité comprend le fait de mettre un étalon interne dans ledit échantillon.

10. Procédé conforme à l'une des revendications précédentes, dans lequel ledit index de polypeptides et de données comporte, pour chacun des polypeptides figurant dans l'index, au moins une caractéristique déterminée de manière empirique.
